# EUROPEAN PATENT SPECIFICATION

(11) **EP 1 012 283 B1**
(45) Date of publication and mention of the grant of the patent: **03.06.2009**
(21) Application number: 98945923.5
(22) Date of filing: 04.09.1998
(51) Int. Cl.: C12N 15/12, C07K 14/705, C07K 19/00, G01N 33/53, G01N 33/68, A61K 38/17, A61K 35/26, C07K 16/30, C12N 5/08

(54) **MAGE-3 PEPTIDES PRESENTED BY HLA CLASS II MOLECULES**
MAGE-3 PEPTIDE, DIE DURCH HLA-KLASSE-II MOLECULE PRESENTIERT WERDEN
PEPTIDES DE MAGE-3 PRESENTES PAR DES MOLECULES DE CLASSE II DE HLA

(30) Priority: 12.09.1997 US 928615
(43) Date of publication of application: 28.06.2000
(73) Proprietor: Ludwig Institute for Cancer Research Ltd., New York, NY 10158 (US); VRIJE UNIVERSITEIT BRUSSEL, 1050 Brussel (BE)
(72) Inventor: THIELEMANS, Kris, B-1090 Brussels (BE); HEIRMAN, Carlo, B-1090 Brussels (BE); CORTHALS, Jurgen, B-1090 Brussels (BE); CHAUX, Pascal, B-1200 Brussels (BE); STROOBANT, Vincent, B-1200 Brussels (BE); BOON-FALLEUR, Thierry, B-1200 Brussels (BE); VAN DER BRUGGEN, Pierre, B-1200 Brussels (BE); LUITEN, Rosalie, B-1200 Brussels (BE)
(74) Representative: Baldock, Sharon Claire
(86) International application number: PCT/US1998/018601
(87) International publication number: WO 1999/014326

(56) References cited:
- WO-A-94/05304
- WO-A-94/23031
- WO-A-95/19783
- WO-A-97/11669
- WO-A-97/13858
- SANDERSON S. ET AL.: "Expression of endogenous peptide-major histocompatibility complex class II complexes derived from invariant chain-antigen fusion proteins" PROCEEDINGS OF THE NATIONAL ACADEMY OF SCIENCES OF USA, vol. 92, no. 16, August 1995, pages 7217-7221, XP002025345 cited in the application
- SPATOLA A.F.: "Peptide backbone modifications: a structure-activity analysis of peptides containing amide bond surrogates" CHEMISTRY AND BIOCHEMISTRY OF AMINO ACIDS, PEPTIDES, AND PROTEINS,1983, pages 267-357, XP002086355
- PLAEN DE E. ET AL.: "Structure, Chromosomal localization, and expression of 12 genes of the MAGE family" IMMUNOGENETICS, vol. 40, no. 5, 1994, pages 360-369, XP000614537 cited in the application
- GAUGLER B. ET AL.: "Human gene MAGE-3 codes for an antigen recognized on a melanoma by autologous cytolytic T lymphocytes" JOURNAL OF EXPERIMENTAL MEDICINE, vol. 179, no. 3, 1 March 1994, pages 921-930, XP002023774 cited in the application
- VAN DER BRUGGEN P. ET AL.: "A peptide encoded by human gene MAGE-3 and presented by HLA-A2 induces cytolytic T lymphocytes that recognize tumor cells expressing MAGE-3" EUROPEAN JOURNAL OF IMMUNOLOGY, vol. 24, no. 12, 1994, pages 3038-3043, XP000614539 cited in the application

## Description

### Field of the Invention

This invention relates to fragments of the tumor associated gene product MAGE-3 which bind to and are presented to T lymphocytes by HLA class II molecules. The peptides, nucleic acid molecules which code for such peptides, as well as related antibodies and CD4⁺T lymphocytes, are useful, *inter alia,* in diagnostic and therapeutic contexts.

### Background of the Invention

The process by which the mammalian immune system recognizes and reacts to foreign or alien materials is complex. An important facet of the system is the T cell response, which in part comprises mature T lymphocytes which are positive for either CD4 or CD8 cell surface proteins. T cells can recognize and interact with other cells via cell surface complexes on the other cells of peptides and molecules referred to as human leukocyte antigens ("HLAs") or major histocompatibility complexes ("MHCs"). The peptides are derived from larger molecules which are processed by the cells which also present the HLA/MHC molecule See Male et al., Advanced Immunology (J.P. Lipincott Company, 1987), especially chapters 6-10. The interaction of T cells and complexes of HLA/peptide is restricted, requiring a specific T cell for a specific complex of an HLA molecule and a peptide. If a specific T cell is not present, there is no T cell response even if its partner complex is present. Similarly, there is no response if the specific complex is absent, but the T cell is present. The mechanisms described above are involved in the immune system's response to foreign materials, in autoimmune pathologies, and in responses to cellular abnormalities.

The T cell response to foreign antigens includes both cytolytic T lymphocytes and helper T lymphocytes. CD8 cytotoxic or cytolytic T cells (CTLs) are T cells which, when activated, lyse cells that present the appropriate antigen presented by HLA class I molecules. CD4⁺ T helper cells are T cells which secrete cytokines to stimulate macrophages and antigen-producing B cells which present the appropriate antigen by HLA class II molecules on their surface.

The mechanism by which T cells recognize alien materials also has been implicated in cancer. A number of cytolytic T lymphocyte (CTL) clones directed against autologous melanoma have been described. In some instances, the antigens recognized by these clones have been characterized. In De Plaen et al., Immunogenetics 40:360-369 (1994), the "MAGE" family. a family of genes encoding tumor specific antigens, is described. (*See also* PCT application PCT/US92/04354, published on November 26, 1992.) The expression products of these genes are processed into peptides which, in turn, are expressed on cell surfaces. This can lead to lysis of the tumor cells by specific CTLs. The genes are said to code for "tumor rejection antigen precursors" or "TRAP" Molecules, and the peptides derived therefrom are referred to as "tumor rejection antigens" or "TRAs". See Traversari et al., Immunogenetics 35: 145 (1992); van der Bruggen et al., Science 254: 1643 (1991), for further information on this family of genes. Also, see U.S. Patent No. 5.342,774.

In U.S. Patent 5.405,940, MAGE nonapeptides are taught which are presented by the HLA-A1 molecule. Given the known specificity of particular peptides for particular HLA molecules, one should expect a particular peptide to bind one HLA molecule, but not others. This is important, because different individuals possess different HLA phenotypes. As a result, while identification of a particular peptide as being a partner for a specific HLA molecule has diagnostic and therapeutic ramifications, these are only relevant for individuals with that particular HLA phenotype. There is a need for further work in the area, because cellular abnormalities are not restricted to one particular HLA phenotype, and targeted therapy requires some knowledge of the phenotype of the abnormal cells at issue.

In U.S. Patent 5,591,430, additional isolated MAGE-3 peptides are taught which arc presented by the HLA-A2 molecule. Therefore, a given TRAP can yield a plurality of TRAs.

The foregoing references describe isolation and/or characterization of tumor rejection antigens which are presented by HLA class I molecules. These TRAs can induce activation and proliferation of CD8' cytotoxic T lymphocytes (CTLs) which recognize tumor cells that express the tumor associated genes (e.g. MAGE genes) which encode the TRAs.

The importance of CD4⁺ T lymphocytes (helper T cells) in antitumor immunity has been demonstrated in animal models in which these cells not only serve cooperative and effector functions, but are also critical in maintaining immune memory (reviewed by Topalian, Currr Opin. Immunol. 6:741-745, 1994). Moreover, several studies support the contention that poor tumor-specific immunity is due to inadequate activation of T helper cells.

It has recently been demonstrated that the tyrosinase gene encodes peptides which are presented by HLA class II molecules to stimulate CD4⁺ T lymphocytes (Topalian et al., 1994; Yee et al., J. Immunol. 157:4079-4086, 1996; Topalian et al.. J. Exp. Med. 183:1965-1971. 1996).

It now has been discovered that the MAGE-3 gene encodes additional tumor rejection antigens which are HLA class II binding peptides. These peptides, when presented by an antigen presenting cell having an HLA class II molecule, effectively induce the activation and proliferation of CD4⁺ T lymphocytes.

WO 94/23031 describes the cloning of the gene encoding the tumor rejection antigen precursor MAGE-3, which is processed to a tumor rejection antigen presented by HLA-A1 molecules.

WO 97/11669 relates to tyrosinase based peptides, which are MHC Class II restricted melanoma antigens.

Sanderson et al (Proc. Natl. Acad. Sci. USA, Vol. 92, pp.7217-7221, August 1995) describes presentation of endogenously synthesized OValbumin or hen egg lysozyme as peptide-MHC Class II complexes when expressed as fusion proteins with the invariant chain (Ii).

The invention is elaborated upon in the disclosure which follows.

The invention provides isolated MAGE-3 peptides which bind HLA class II molecules and comprise the amino acid sequence of SEQ ID NO:11 and are a fragment of the amino acid sequence SEQ ID NO: 2. The invention also provides isolated nucleic acid molecules encoding such peptides, expression vectors containing those nucleic acid molecules, host cells transfected with those nucleic acid molecules, and antibodies to those peptides T lymphocytes which recognize complexes of the peptides and HLA class II antigen presenting molecules are also provided. Compositions containing the foregoing molecules additionally are provided. The foregoing can be used in the diagnosis or treatment of conditions characterized by the expression of MAGE-3.

According to one aspect of the invention, an isolated MAGE-3 HLA class II-binding peptide, comprising a fragment of the amino acid sequence of SEQ ID NO:2 which binds an HLA class II molecule and comprises the amino acid sequence of SEQ ID NO: 11 is provided. In certain embodiments, the isolated HLA class II-binding peptide comprises an amino acid sequence selected from the group consisting of SEQ ID NO:3, SEQ ID NO:4, SEQ ID NO:9, and SEQ ID NO: 10. In preferred embodiments, the isolated peptide consists of an amino acid sequence selected from the group consisting of SEQ ID NO:3, SEQ ID NO:4, SEQ ID NO:9, SEQ ID NO: 10 and SEQ ID NO: 11. More preferably, the isolated peptide consists of an amino acid sequence selected from the group consisting of SEQ ID NO:3 and SEQ ID NO:4. In certain embodiments, the isolated peptide further comprises an endosomal targeting signal, preferably an endosomal targeting portion of human invariant chain Ii. In other embodiments of the invention, the isolated HLA class II-binding peptide is non-hydrolyzable. Preferred non-hydrolyzable peptides are selected from the group consisting of peptides comprising D-amino acids, peptides comprising a -psi[CH₂NH]-reduced amide peptide bond, peptides comprising a -psi[COCH₂]-ketomethylene peptide bond, peptides comprising a -psi[CH(CN)NH]-(cyanomethylene)amino peptide bond, peptides comprising a - psi[CH₂ CH(OH)]-hydroxyethylene peptide bond, peptides comprising a -psi[CH,O]-peptide bond, and peptides comprising a -psi[CH₂SHomethylene peptide bond.

According to another aspect of the invention, a composition comprising an isolated MAGE-3 HLA class I-binding peptide and an isolated MAGE-3 HLA class II-binding peptide of the invention is provided. In certain embodiments,the MAGE-3 HLA class I-binding peptide and the MAGE-3 HLA class II-binding peptide are combined as a polytope polypeptide. The isolated MAGE-3 HLA class peptide in the composition comprises the amino acid sequence of SEQ ID NO: 11 and is a fragment of the amino acid sequence of SEQ ID NO:2. Preferably, the isolated IMAGE-3 HLA class II-binding peptide in the composition consists of an amino acid sequence selected from the group consisting of SEQ ID NO:3, SEQ ID NO:4, SEQ ID NO:9, SEQ ID NO:10 and SEQ ID NO:11. More preferably, the isolated MAGE-3 HLA class II-binding peptide consists of an amino acid sequence selected from the group consisting of SEQ ID NO:3 and SEQ ID NO:4. In certain embodiments of the foregoing compositions, the isolated MAGE-3 HLA class II-binding peptide includes an endosomal targeting signal. Preferably the endosomal targeting signal includes an endosomal targeting portion of human invariant chain Ii.

According to another aspect of the invention, an isolated nucleic acid encoding any of the foregoing HLA class II-binding peptides is provided. Preferably the nucleic acid comprises SEQ ID NO:13

According to still another aspect of the invention, expression vectors are provided. The expression vectors comprise any of the foregoing isolated nucleic acids operably linked to a promoter. In preferred embodiments, the nucleic acid comprises SEQ ID NO:13. In other embodiments, the expression vector further comprise a nucleic acid which encodes an HLA-DRB 1/13 molecule.

According to yet another aspect of the invention, host cells transfected or transformed with any of the foregoing expression vectors are provided. Host cells which express an HLA-DRB 1/13 molecule, and which are transfected or transformed with any of the foregoing expression vectors are also provided.

According to another aspect of the invention, methods for enriching selectively a population of T lymphocytes with CD4⁺ T lymphocytes specific for a MAGE-3 HLA class II-binding peptide of the invention are provided. The methods include contacting an isolated population of T lymphocytes with an isolated antigen presenting an which comprises a complex of the MAGE-3 HLA class II-binding peptide and an HLA class II molecule in an amount sufficient to selectively enrich the isolated population of T lymphocytes with the CD4⁺ T lymphocytes. In preferred embodiments, the HLA class II molecule is an HLA-DRB 1/13 molecule and the MAGE-3 HLA class II-binding peptide is selected from the group consisting of a peptide consisting of the amino acid sequence of SEQ ID NO:3, a peptide consisting of the amino acid sequence of SEQ ID NO:4, peptide consisting of the amino acid sequence of SEQ ID NO:9, peptide consisting of the amino acid sequence of SEQ ID NO: 10, and a peptide consisting of the amino acid sequence of SEQ ID NO: 11. More preferably, the MAGE-3 HLA class II-binding peptide is selected from the group consisting of a peptide consisting of the amino acid sequence of SEQ ID NO:3 and a peptide consisting of the amino acid sequence of SEQ ID NO:4. In certain embodiments of the foregoing methods, the isolated MAGE-3 protein or HLA class II binding peptide thereof includes an endosomal targeting signal. Preferably the endosomal targeting signal includes an endosomal targeting portion of human invariant chain Ii.

According to a further aspect of the invention, methods for diagnosing a disorder characterized by expression of MAGE-3 are provided. The methods include contacting a biological sample isolated from a subject with an antibody of the invention that is specific for AGE-3 class II binding peptide of the invention and determining the interaction between the antibody and the MAGE-3 HLA class II binding peptide as a determination of the disorder. In preferred embodiments, the MAGE-3 HLA class II-binding peptide is selected from the group consisting of a peptide consisting of the amino acid sequence of SEQ ID NO:3, a peptide consisting of the amino acid sequence of SEQ ID NO:4, peptide consisting of the amino acid sequence of SEQ ID NO:9, peptide consisting of the amino acid sequence of SEQ ID NO:10, and a peptide consisting of the amino acid sequence of SEQ ID NO:1 1. More preferably, the MAGE-3 HLA class 11-binding peptide is selected from the group consisting of a peptide consisting of the amino acid sequence of SEQ ID NO:3 and a peptide consisting of the amino acid sequence of SEQ ID NO:4.

According to another aspect of the invention, methods for diagnosing a disorder characterized by expression of a MAGE-3 HLA class II-binding peptide of the invention which forms a complex with an HLA class II molecule are provided. The methods include contacting a biological sample isolated from a subject with an isolated CD4⁺T lymphocyte of the invention that binds the complex, and determining binding between the complex and the lymphocyte a determination of the disorder. In some embodiments the HLA class II molecule is an HLA-DRB1/13 molecule, such as HLA-DRB1/1301 or HLA-DRB1/1302. Preferably the MAGE-3 HLA class II-binding peptide is selected from the group consisting of a peptide consisting of the amino acid sequence of SEQ ID NO:3, a peptide consisting of the amino acid sequence of SEQ ID NO:4, peptide consisting of the amino acid sequence of SEQ ID NO:9, peptide consisting of the amino acid sequence of SEQ ID NO:10, and a peptide consisting of the amino acid sequence of SEQ ID NO:11. More preferably, the MAGE-3 HLA class II-binding peptide is selected from the group consisting of a peptide consisting of the amino acid sequence of SEQ ID NO:3 and a peptide consisting of the amino acid sequence of SEQ ID NO:4.

The invention also provides a MAGE-3 HLA Class II - binding peptide of the invention, an expression vector of the invention, a cell of the invention or an isolated autologous CD4⁺ T lymphocyte of the invention for use in therapy. In specific embodiments, the HLA class II molecule is an HLA-DRB1/13 molecule, such as HLA-DRB1/1301 or HLA-DRB 1/1302.

According to another aspect of the invention, an antibody is provided which binds selectively a MAGE-3 HLA class II-binding peptide of the invention In certain embodiments, the antibody is a monoclonal antibody. In other embodiments, the antibody is an antibody fragment selected from the group consisting of a Fab fragment, a F(ab)₂ fragment or a fragment including a CDR3 region selective for a MAGE-3 HLA class II-binding peptide.

According to still another aspect of the invention, an isolated CD4⁺ T lymphocyte is provided. The isolated CD4⁺ T lymphocyte selectively binds a complex of an HLA class II molecule and a MAGE-3 HLA class II-binding peptid of the invention. Preferably the HLA class II molecule is an HLA-DRB1/13 molecule. Preferably the MAGE-3 HLA class II-binding peptide is selected from the group consisting of a peptide consisting of the amino acid sequence of SEQ ID NO:3, a peptide consisting of the amino acid sequence of SEQ IDNO:4, peptide consisting of the amino acid sequence of SEQ ID NO:9. peptide consisting of the amino acid sequence of SEQ ID NO:10, and a peptide consisting of the amino acid sequence of SEQ ID NO:11. More preferably, the MAGE-3 HLA class 11-binding peptide is selected from the group consisting of a peptide consisting of the amino acid sequence of SEQ ID NO:3 and a peptide consisting of the amino acid sequence of SEQ ID NO:4.

Methods for identifying functional variants of a MAGE-3 HLA class II binding peptide of the invention are provided according to another aspect of the invention. According to the methods, a MAGE-3 HLA class II binding peptide, an HLA class II binding molecule which binds the MAGE-3 HLA class II binding peptide, and a T cell which is stimulated by the MAGE-3 HLA class II binding peptide presented by the HLA class II binding molecule are selected. A first amino acid residue of the MAGE-3 HLA class II binding peptide is mutated to prepare a variant peptide. The binding of the variant peptide to HLA class II binding molecule and stimulation of the T cell are then determined, wherein binding of the variant peptide to the HLA class II binding molecule and stimulation of the T cell by the variant peptide presented by the HLA class II binding molecule indicates that the variant peptide is a functional variant. Preferably, the peptide consists of the amino acid sequence of SEQ ID NO:3. SEQ ID NO:4, SEQ ID NO:9, SEQ ID NO:10, or SEQ ID NO: 11. In certain embodiments, the methods further include the step of comparing the stimulation of the T cell by the MAGE-3 HLA class II binding peptide and the stimulation of the T cell by the functional variant as a determination of the effectiveness of the stimulation of the T cell by the functional variant.

The invention also provides pharmaceutical preparations containing any one or more of the peptides of the invention. Such pharmaceutical preparations can include pharmaceutically acceptable diluent carriers or excipients.

These and other objects of the invention will be described in further detail in connection with the detailed description of the invention.

### Brief Description of the Drawings

Figure 1 is a schematic representation of the protocol used to obtain CD4 T cell lines specific for MAGE-3.
Figure 2 is a graph showing CD4⁺ T cell lines B6 and F3 recognized autologous EBV-B cells which have processed the recombinant His-MAGE-3 protein.
Figure 3 is a graph showing that the recognition by CD4⁺ T cell clones of autologous EBV-B cells pulsed with exogenous His-MAGE-3 protein is inhibited by an anti-HLA DR monoclonal antibody.
Figure 4 is a graph detailing the screening of MAGE-3 peptides for recognition by CD4* clones B6/34, B6/37, F3/37 and F3/40.
Figure 5 is a graph depicting stimulation of TNF and IFN-y production by CD4⁺ clones B6/34 and B6/37 EBV-B cells pulsed with the peptide RKVAELVHFLLLKYRA (MAGE-3,,,. 126, SEQ ID NO:3) or ELVHFLLLKYRAREPV (MAGE-3₁₁₅₋₁₃₀, SEQ ID NO:4).
Figure 6 is a graph which shows that autologous EBV-B cells pulsed with the peptide MAGE-3₁₁₁₋₁₂₆ or MAGE-3₁₁₅₋₁₃₀ induced the proliferation of clones B6/34 and B6/37.

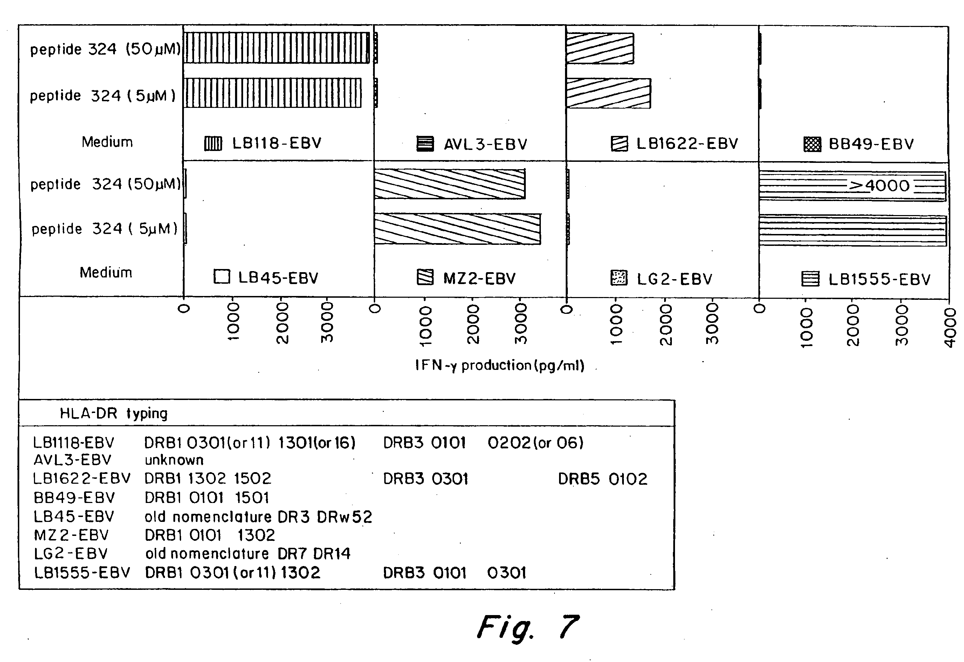

Figure 7 is a graph which demonstrates that the response of CD4⁺ clone B6/37 to peptide MAGE-3₁₁₅₋₁₃₀ is HLA-DRBI/1302 restricted.
Figure 8 is a graph which shows the reactivity of clone B6/37 against autologous EBV-B cells pulsed with truncated peptides derived from MAGE-3₁₁₅₋₁₃₀.
Figure 9 shows the recognition of transduced MZ2 EBV by CD4 T cell clone 426/B6.37 (anti -MAGE-3.DR13).
Figure. 10 shows the recognition of transduced MZ2 EBV by CTL clone 434/1 (anti-MAGE-3.A1).
Figure 11 shows the lysis of transduced MZ2 EBV by CTL434/1 (anti-MAGE-3.A1).
Figures 12A and 12 B show the recognition of transduced MZ2-MEL.43 by CD4 T cell clone 426/B6.37 (anti-MAGE-3.DR13) and CTL clone 434/1 (anti-MAGE-3.A1), respectively.

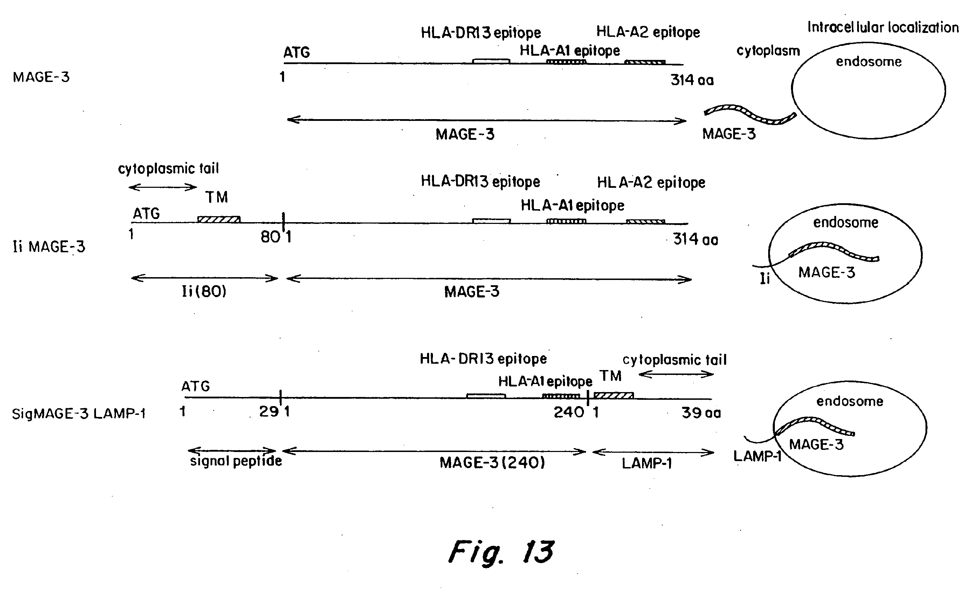

Figure 13 is a schematic drawing of retroviral constructs of invariant chain (Ii) and LAMP-1 MANGE-3 fusion proteins.

### Detailed Description of the Invention

The invention provides isolated MAGE-3 peptides presented by HLA class II molecules, which peptides stimulate the proliferation and activation of Cod4* T lymphocytes. Such peptides are referred to herein as "MAGE-3 HLA class II binding peptides" and "HLA class II binding peptides". Hence, one aspect of the invention is an isolated peptide which includes the amino acid sequence of SEQ ID NO: 11 and that is a fragment of the amino acid sequence of SEQ ID NO:2.

The examples below show the isolation of peptides which are MAGE-3 HLA class II binding peptides. These exemplary peptides are processed translation products of the nucleic acid of SEQ ID NO:1. As such, it will be appreciated by one of ordinary skill in the art that the translation products from which a MAGE-3 HLA class II binding peptide is processed to a final form for presentation may be of any length or sequence so long as they encompass the MAGE-3 HLA class II binding peptide. As demonstrated in the examples below, peptides or proteins as small as 10 amino acids and as large as the amino acid sequence of the MAGE-3 protein ID NO:2) are appropriately processed, presented by HLA class II molecules and effective in stimulating CD4⁺ T lymphocytes. MAGE-3 HLA class II binding peptides, such as the peptide of SEQ ID NO:11, may have one, two, three, four, five, six, seven, eight, nine, ten, or more amino acids added to either or both ends. The antigenic portion of such a peptide is cleaved out under physiological conditions for presentation by HLA class II molecules. Additional MAGE-3 HLA class II binding peptides, as well as MAGE family HLA class II. binding peptides, can be identified by one of ordinary skill in the art according to the procedures described herein.

The procedures described in the Examples can be utilized to identify MAGE family HLA class II binding peptides. Thus, for example, one can load antigen presenting cells, such as dendritic cells of normal blood donors, with a recombinant MAGE protein (or a fragment thereof) by contacting the cells with the MAGE polypeptide or by introducing into the cells a nucleic acid molecule which directs the expression of the MAGE protein of interest. The antigen-presenting cells then can be used to induce *in vitro* the activation and proliferation of specific CD4 lymphocytes which recognize MAGE HLA class II binding peptides. The sequence of the peptides then can be determined as described in the Examples, e.g.. by stimulating cells with peptide fragments of the MAGE protein used to stimulate the activation and proliferation of CD4 lymphocytes. Alternatively, one can load antigen presenting cells with peptides derived from a MAGE protein. For example, one can make predictions of peptide sequences derived from MAGE family proteins which are candidate HLA class II binding peptides based on the consensus amino acid sequences for binding HLA class II molecules. In this regard, see, e.g. International applications PCT/US96/03182 and PCT/US98/01373. Peptides which are thus selected can be used in the assays described herein for inducing specific CD4 lymphocytes and identification of peptides. Additional methods of selecting and testing peptides for HLA class II binding are well known in the art.

As used herein, a "functional variant" or "variant" of a HLA class II binding peptide is a peptide which contains one or more modifications to the primary amino acid sequence of a HLA class II binding peptide and retains the HLA class II and T cell receptor binding properties disclosed herein. Modifications which create a MAGE-3 HLA class II binding peptide functional variant can be made for example 1) to enhance a property of a MAGE-3 HLA class II binding peptide, such as peptide stability in an expression system or the stability of protein-protein binding such as HLA-peptide binding; 2) to provide a novel activity or property to a MAGE-3 HLA class II binding peptide, such as addition of an antigenic epitope or addition of a detectable moiety; or 3) to provide a different amino acid sequence that produces the same or similar T cell stimulatory properties. Modifications to MAGE-3 (as well as MAGE family) HLA class II binding peptides can be made to nucleic acids which encodes the peptide, and can include deletions, point mutations, truncations, amino acid substitutions and additions of amino acids. Alternatively, modifications can be made directly to the polypeptide, such as by cleavage, addition of a linker molecule, addition of a detectable moiety, such as biotin, addition of a fatty acid, substitution of one amino acid for another and the like. Variants also can be selected from libraries of peptides, which can be random peptides or peptides based on the sequence of the MAGE peptides including subtitutions at one or more positions. For example, a peptide library can be used in competition assays with complexes of MAGE peptides bound to HLA class II molecules (e.g. dendritic cells loaded with MAGE peptide). Peptides which compete for binding of the MAGE peptide to the HLA class II molecule can be sequenced and used in other assays (e.g. CD4 lymphocyte proliferation) to determine suitability as MAGE peptide functional variants.

Modifications also embrace fusion proteins comprising all or part of a MAGE HLA class II binding peptide amino acid sequence, such as the invariant chain-MAGE-3 fusion proteins described herein. The invention thus embraces fusion proteins comprising MAGE-3 HLA class II binding peptides and endosomal targeting signals such as the human invariant chain (Ii). As is disclosed below, fusion of an endosomal targeting portion of the human invariant chain to MAGE-3 resulted in efficient targeting of MAGE-3 to the HLA class II peptide presentation pathway. An "endosomal targeting portion" of the human invariant chain or other targeting polypeptide is that portion of the molecule which, when fused or conjugated to a second polypeptide, increases endosomal localization of the second polypeptide. Thus endosomal targeting portions can include the entire sequence or only a small portion of a targeting polypeptide such as human invariant chain Ii. One of ordinary skill in the art can readily determine an endosomal targeting portion of a targeting molecule.

Surprisingly, fusion of an endosomal targeting portion of LAMP-1 protein did not significantly increase targeting of MAGE-3 to the HLA class II peptide presentation pathway. Therefore, the invention includes the unexpected finding that fusion proteins of MAGE-3 and human invariant chain Ii, but not LAMP-1, are efficiently targeted to the HLA class II peptide presentation pathway. Additional endosomal targeting signals can be identified by one of ordinary skill in the art, fused to MAGE-3 or a MAGE-3 HLA class II binding portion thereof, and tested for targeting to the HLA class II peptide presentation pathway using no more than routine experimentation.

The amino acid sequence of MAGE HLA class II binding peptides may be of natural or non-natural origin, that is, they may comprise a natural MAGE HLA class II binding peptide molecule or may comprise a modified sequence as long as the amino acid sequence retains the ability to stimulate helper T cells when presented and retains the property of binding to an HLA class II molecule such as an HLA DRBI/13 molecule. For example, MAGE-3 HLA class II binding peptides in this co text may be fusion proteins including a MAGE-3 HLA class II binding peptide as defined unrelated amino acid sequences, synthetic peptides of amino acid sequences shown in SEQ ID Nos:3,4, 9,10 and 11, labeled peptides, peptides isolated from patients with a MAGE-3 expressing cancer, peptides isolated from cultured cells which express MAGE-3, peptides coupled to nonpeptide molecules (for example in certain drug delivery systems) and other molecules which include the amino acid sequence of SEQ ID NO:11 and are a fragment of the amino acid sequence of SEQ ID NO: 2.

Preferably, MAGE HLA class II binding peptides are non-hydrolyzable. To provide such peptides, one may select MAGE HLA class 11 binding peptides from a library of non-hydrolyzable peptides, such as peptides containing one or more D-amino acids or peptides containing one or more non-hydrolyzable peptide bonds linking amino acids. Alternatively, one can select peptides which are optimal for inducing CD4 T lymphocytes and then modify such peptides as necessary to reduce the potential for hydrolysis by proteases. For example, to determine the susceptibility to proteolytic cleavage, peptides may be labeled and incubated with cell extracts or purified proteases and then isolated to determine which peptide bonds are susceptible to proteolysis, e.g., by sequencing peptides and proteolytic fragments. Alternatively, potentially susceptible peptide bonds can be identified by comparing the amino acid sequence of a MAGE-3 HLA class II binding peptide with the known cleavage site specificity of a panel of proteases. Based on the results of such assays, individual peptide bonds which are susceptible to proteolysis can be replaced with non-hydrolyzable peptide bonds by *in vitro* synthesis of the peptide.

Many non-hydrolyzable peptide bonds are known in the art, along with procedures for synthesis of peptides containing such bonds. Non-hydrolyzable bonds include -psi[CH₂;)NH]-reduced amide peptide bonds, -psi[COCH₂]- ketomethylene peptide bonds, -psi[CH(CN)NH]-(cyanomethylene)amino peptide bonds, -psi[CH,CH(OH)]- hydroxyethylene peptide bonds, -psi[CH₂O]- peptide bonds, and -psi[CH₂S]- thiomethylene peptide bonds.

Nonpeptide analogs of peptides, e.g., those which provide a stabilized structure or lessened biodegradation, are also contemplated. Peptide mimetic analogs can be prepared based on a selected MAGE-3 HLA class II binding peptide by replacement of one or more residues by nonpeptide moieties. Preferably, the nonpeptide moieties permit the peptide to retain its natural conformation, or stabilize a preferred, e.g., bioactive, confirmation. Such peptides can be tested in molecular or cell-based binding assays to assess the effect of the substitution(s) on conformation and/or activity. One example of methods for preparation of nonpeptide mimetic analogs from peptides is described in Nachman et al., Regul. Pept. 57:359-370 (1995). Peptide as used herein embraces all of the foregoing.

If a variant involves a change to an amino acid of SEQ ID NO:3.. SEQ ID NO:4, SEQ ID NO:9., SEQ ID NO: 10 or SEQ ID NO:11. functional variants of the MAGE-3 HLA class II binding peptide having conservative amino acid substitutions typically will be preferred, i.e., substitutions which retain a property of the original amino acid such as charge, hydrophobicity, conformation, etc. Examples of conservative substitutions of amino acids include substitutions made amongst amino acids within the following groups: (a) M.1. L. V; (b) F. Y. W: (c) K, R. H: (d) A, G; (e) S, T; (f) Q, N; and (g) E. D.

Other methods for identifying functional variants of the MANGE-3 HLA class II binding peptides are provided in a published PCT application of Strominger and Wucherpfennig (PCT/US96/03182). These methods rely upon the development of amino acid sequence motifs to which potential epitopes may be compared. Each motif describes a finite set of amino acid sequences in which the residues at each (relative) position may be (a) restricted to a single residue, (b) allowed to vary amongst a restricted set of residues, or (c) allowed to vary amongst all possible residues. For example, a motif might specify that the residue at a first position may be any one of the residues valine, leucine, isoleucine, methionine, or phenylalanine: that the residue at the second position must be histidine: that the residue at the third position may be any amino acid residue; that the residue at the fourth position may be any one of the residues valine, leucine, isoleucine, methionine, phenylalanine, tyrosine or tryptophan; and that the residue at the fifth position must be lysine.

Sequence motifs for MAGE-3 HLA class II binding peptide functional variants can be developed by analysis of the binding domains or binding pockets of major histocompatibility complex HLA-DR proteins and/or the T cell receptor ("TCR") contact points of the MAGE-3 HLA class II binding peptides disclosed herein. By providing a detailed structural analysis of the residues involved in forming the IILA class II binding pockets, one is enabled to make predictions of sequence motifs for binding of MAGE peptides to any of the HLA class II proteins.

Using these sequence motifs as search, evaluation, or design criteria, one is enabled to identify classes of peptides (e.g. MAGE HLA class II binding peptides, particularly the MAGE-3 peptides disclosed herein, and functional variants thereof) which have a reasonable likelihood of binding to a particular HLA molecule and of interacting with a T cell receptor to induce T cell response. These peptides can be synthesized and tested for activity as described herein. Use of these motifs, as opposed to pure sequence homology (which excludes many peptides which are antigenically similar but quite distinct in sequence) or sequence homology with unlimited "conservative" substitutions (which admits many peptides which differ at critical highly conserved sites), represents a method by which one of ordinary skill in the art can evaluate peptides for potential application in the treatment of disease.

The Strominger and Wucherpfennig PCT application, and references cited therein, describe the HLA class II and TCR binding pockets which contact residues of an HLA class II peptide. By keeping the residues which are likely to bind in the HLA class II and/or TCR binding pockets constant or permitting only specified substitutions, functional variants of MAGE HLA class II binding peptides can be prepared which retain binding to HLA class II and T cell receptor.

Thus methods for identifying additional MAGE family HLA class II peptides, in particular MAGE-3 HLA class II binding peptides, and functional variants thereof, are provided. In general, any MAGE protein can be subjected to the analysis noted above, peptide sequences selected and the tested as described herein. With respect to MAGE-3, for example, the methods include selecting a MAGE-3 HLA class II binding peptide, an HLA class II binding molecule which binds the MAGE-3 HLA class II binding peptide, and a T cell which is stimulated by the MAGE-3 HLA class II binding peptide presented by the HLA class II binding molecule. In preferred embodiments, the MAGE-3 HLA class II binding peptide consists of the amino acid sequence of SEQ ID NO:3, SEQ ID NO:4, SEQ ID NO:9, SEQ ID NO:10, or SEQ ID NO:11. A first amino acid residue of the MAGE-3 HLA class II binding peptide is mutated to prepare a variant peptide. The amino acid residue can be mutated according-to the principles of HLA and T cell receptor contact points set forth in the Strominger and Wucherpfennig PCT application described above. Any method for preparing variant peptides can be employed, such as synthesis of the variant peptide, recombinantly producing the variant peptide using a mutated nucleic acid molecule, and the like.

The binding of the variant peptide to HLA- class II binding molecule and stimulation of the T cell are then determined according to standard procedures. For example, as exemplified below, the variant peptide can be contacted with an antigen presenting cell which contains the HLA class II molecule which binds the MAGE-3 peptide to form a complex of the variant peptide and antigen presenting cell. This complex can then be contacted with a T cell which recognizes the MAGE-3 HLA class II binding peptide presented by the HLA class II binding molecule. T cells can be obtained from a patient having a condition characterized by expression of MAGE-3. Recognition of variant peptides by the T cells can be determined by measuring an indicator of T cell stimulation such as TNF or IFNγ production. Similar procedures can be carried out for identification and characterization of other MAGE family HLA class II binding peptides.

Binding of a variant peptide to the HLA class II binding molecule and stimulation of the T cell by the variant peptide presented by the HLA class II binding molecule indicates that the variant peptide is a functional variant. The methods also can include the step of comparing the stimulation of the T cell by the MAGE-3 HLA class II binding peptide and the stimulation of the T cell by the functional variant as a determination of the effectiveness of the stimulation of the T cell by the functional variant. By comparing the functional variant with the MAGE-3 HLA class II binding peptide, peptides with increased T cell stimulatory proterties can be prepared.

Variants of the MAGE-3 HLA class II binding peptides prepared by any of the foregoing methods can be sequenced, if necessary, to determine the amino acid sequence and thus deduce the nucleotide sequence which encodes such variants.

Also a part of the invention are those nucleic acid sequences which code for a MAGE HLA class II binding peptide of the invention. The term "stringent conditions" as used herein refers to parameters with which the art is familiar. Nucleic acid hybridization parameters may be found in references which compile such methods, e.g. Molecular Cloning: A Laboratory Manual, J. Sambrook, et al., eds., Second Edition, Cold Spring Harbor Laboratory Press, Cold Spring Harbor, New York, 1989, or Current Protocols in Molecular Biology, F.M. Ausubel, et al., eds., John Wiley & Sons, Inc., New York. More specifically, stringent conditions, as used herein, refers to hybridization at 65°C in hybridization buffer (3.5 x SSC, 0.02% Ficoll, 0.02% Polyvinyl pyrolidone, 0.02% Bovine Serum Albumin, 25mMNaH₂PO₄ (pH7), 0.5% SDS, 2mM EDTA). SSC is 0.15M Sodium Chloride/0.15M Sodium Citrate, pH 7; SDS is Sodium Dodecyl Sulphate; and EDTA is Ethylene diaminetetraacetic acid. After hybridization, the membrane upon which the DNA is transferred is washed at 2xSSC at room temperature and then at 0.1xSSC/0.1xSDS at 65°C.

There are other conditions, reagents, and so forth which can used, which result in a similar degree of stringency. The skilled artisan will be familiar with such conditions, and thus they are not given here. It will be understood, however, that the skilled artisan will be able to manipulate the conditions in a manner to permit the clear identification of homologs and alleles of nucleic acids encoding the MAGE HLA class II binding peptides of the invention. The skilled artisan also is familiar with the methodology for screening cells and libraries for expression of such molecules which then are routinely isolated, followed by isolation of the pertinent nucleic acid molecule and sequencing.

In general homologs and alleles typically will share at least 50% amino acid identity and/or at least 40% nucleotide identity to the amino acid sequence of a MAGE-3 HLA class II binding peptide (such as SEQ ID NOs:3, 4, 9, 10 or 11) or nucleic acids which encode such a peptide, respectively. In some instances homologs and alleles will share at least 50% nucleotide identity and/or at least 65% amino acid identity and in still other instances will share at least 60% nucleotide identity and/or at least 75% amino acid identity. Complements of the foregoing nucleic acids also are envisaged.

In screening for nucleic acids which encode a MAGE HLA class II binding peptide, a nucleic acid hybridization such as a Southern blot or a Northern blot may be performed using the foregoing conditions, together with a ³²P probe. After washing the membrane to which DNA encoding a MAGE HLA class II binding peptide was finally transferred, the membrane can be placed against X-ray film to detect the radioactive signal.

The invention also includes the use of nucleic acid sequences which include alternative codons that encode the same amino acid residues of the MAGE HLA class II binding peptides. For example, as disclosed herein, the peptide RKVAELVHFLLLKYRA (SEQ ID NO:3) is a MAGE-3 HLA class II binding peptide. The leucine residues (amino acids No. 6, 10, 11 and 12 of SEQ ID NO:3) can be encoded by the codons CUA, CUC, CUG, CUU, UUA and UUG. Each of the six codons is equivalent for the purposes of encoding a leucine residue. Thus, it will be apparent to one of ordinary skill in the art that any of the leucine-encoding nucleotide triplets may be employed to direct the protein synthesis apparatus, *in vitro* or *in vivo,* to incorporate a leucine residue. Similarly, nucleotide sequence triplets which encode other amino acid residues comprising the MAGE-3 HLA class II binding peptide of SEQ ID NO:3 include: CGA, CGC, CGG, CGT, AGA and AGG (arginine codons); AAA and AAG (lysine codons); GUA, GUC, GUG and GUU (valine codons); GAA and GAG (glutamine codons); CAC and CAU (histidine codons); UUC and UUU (phenylalanine codons) andUAC and UAU (tyrosine codons). Other amino acid residues may be encoded similarly by multiple nucleotide sequences. Thus, the invention embraces degenerate nucleic acids that differ from the native MAGE HLA class II binding peptide encoding nucleic acids in codon sequence due to the degeneracy of the genetic code.

It will also be understood that the invention embraces the use of the sequences in expression vectors, as well as to transfect host cells and cell lines, be these prokaryotic (e.g., *E. coli*)*,* or eukaryotic (e.g., dendritic cells, CHO cells. COS cells, yeast expression systems and recombinant baculovirus expression in insect cells). The expression vectors require that the pertinent sequence, i.e., those described *supra,* be operably linked to a promoter. As it has been found that human HLA-DRB1/1302 molecules present a MAGE-3 HLA class II binding peptide, the expression vector may also include a nucleic acid sequence coding for an HLA-DRB 1/13 molecule. (For other MAGE HLA class II binding peptides, different HLA molecules can be used.) In a situation where the vector contains both coding sequences, it can be used to transfect a cell which does not normally express either one. The MANGE-3 HLA class II binding peptide coding sequence may be used alone, when, e.g. the host cell already expresses an HLA-DRB 1/13 molecule. Of course, there is no limit on the particular host cell which can be used as the vectors which contain the two coding sequences may be used in host cells which do not express HLA-DRB1/13 molecules if desired, and the nucleic acid coding for the MAGE-3 HLA class II binding peptide can be used in antigen presenting cells which express an HLA-DRB1/13 molecule. As used herein, "an HLA-DRB 1/13 molecule" includes the subtypes DRB1*1301, DRB1*1302, DRB1*13031, DRB1*13032, DRB1*1304, DRB1*1305, DRB1*1306, DRB1*1307, DRB1*1308, DRB1*1309, DRB1*1310, DRB1*1311, DRB1*1312, DRB1*1314, DRB1*1315, DRB1*1316, DRB1*1317, DRB1*1318, DRB1*1319, DRB1*1320, DRB1*1321, DRB1* 1322, DRB1*1323 and DRB 1* 1324.

As used herein, a "vector" may be any of a number of nucleic acids into which a desired sequence may be inserted by restriction and ligation for transport between different genetic environments or for expression in a host cell. Vectors are typically composed of DNA although RNA vectors are also available. Vectors include, but are not limited to, plasmids, phagemids and virus genomes. A cloning vector is one which is able to replicate in a host cell, and which is further characterized by one or more endonuclease restriction sites at which the vector may be cut in a determinable fashion and into which a desired DNA sequence may be ligated such that the new recombinant vector retains its ability to replicate in the host cell. In the case of plasmids, replication of the desired sequence may occur many times as the plasmid increases in copy number within the host bacterium or just a single time per host before the host reproduces by mitosis. In the case of phage, replication may occur actively during a lytic phase or passively during a lysogenic phase. An expression vector is one into which a desired DNA sequence may be inserted by restriction and ligation such that it is operably joined to regulatory sequences and may be expressed as an RNA transcript. Vectors may further contain one or more marker sequences suitable for use in the identification of cells which have or have not been transformed or transfected with the vector. Markers include, for example, genes encoding proteins which increase or decrease either resistance or sensitivity to antibiotics or other compounds, genes which encode enzymes whose activities are detectable by standard assays known in the art (e.g.. β-galactosidase or alkaline phosphatase), and genes which visibly affect the phenotype of transformed or transfected cells, hosts, colonies or plaques (e.g., green fluorescent protein). Preferred vectors are those capable of autonomous replication and expression of the structural gene products present in the DNA segments to which they are operably joined.

Preferably the expression vectors contain sequences which target a MAGE family polypeptide, e.g. MAGE-3, or a HLA class II binding peptide derived therefrom, to the endosomes of a cell in which the protein or peptide is expressed. HLA class II molecules contain an invariant chain (Ii) which impedes binding to other molecules to the HLA class II molecules. This invariant chain is cleaved in endosomes, thereby permitting binding of peptides by HLA class II molecules. Therefore it is preferable that the MAGE-3 HLA class II binding peptides and precursors thereof (e.g. the MAGE-3 protein) are targeted to the endosome, thereby enhancing MAGE-3 HLA class II binding peptide binding to HLA class II molecules. Targeting signals for directing molecules to endosomes are know in the art and these signals conveniently can be incorporated in expression vectors such that fusion proteins which contain the endosomal targeting signal are produced. Sanderson et al. (Proc. Nat'l. Acad. Sci. USA 92:7217-7221, 1995), Wu et al. (Proc. Nat'l. Acad. Sci. USA 92:11671-11675, 1995) and Thomson et al (J. Virol. 72:2246-2252. 1998) describe endosomal targeting signals (including invariant chain Ii and lysosomal-associated membrane protein LAMP-1) and their use in directing antigens to endosomal and/or lysosomal cellular compartments. As disclosed in the Examples, invariant chain-MAGE-3 fusion proteins are preferred.

Endosomal targeting signals such as invariant chain also can be conjugated to MAGE-3 protein or peptides by non-peptide bonds (i.e. not fusion proteins) to prepare a conjugate capable of specifically targeting MAGE-3. Specific examples of covalent bonds include those wherein bifunctional cross-linker molecules are used. The cross-linker molecules may be homobifunctional or heterobifunctional, depending upon the nature of the molecules to be conjugated. Homobifunctional cross-linkers have two identical reactive groups. Heterobifunctional cross-linkers are defined as having two different reactive groups that allow for sequential conjugation reaction. Various types of commercially available cross-tinkers are reactive with one or more of the following groups: primary amines, secondary amines, sulfhydryls, carboxyls, carbonyls and carbohydrates. One of ordinary skill in the art will be able to ascertain without undue experimentation the preferred molecule for linking the endosomal targeting moiety and MANGE-3 peptide or protein, based on the chemical properties of the molecules being linked and the preferred characteristics of the bond or bonds.

As used herein, a coding sequence and regulatory sequences are said to be "operably" joined when they are covalently linked in such a way as to place the expression or transcription of the coding sequence under the influence or control of the regulatory sequences. If it is desired that the coding sequences be translated into a functional protein, two DNA sequences are said to be operably joined if induction of a promoter in the 5' regulatory sequences results in the transcription of the coding sequence and if the nature of the linkage between the two DNA sequences does not (1) result in the introduction of a frame-shift mutation, (2) interfere with the ability of the promoter region to direct the transcription of the coding sequences, or (3) interfere with the ability of the corresponding RNA transcript to be translated into a protein. Thus, a promoter region would be operably joined to a coding sequence if the promoter region were capable of effecting transcription of that DNA sequence such that the resulting transcript might be translated into the desired protein or polypeptide.

The precise nature of the regulatory sequences needed for gene expression may vary between species or cell types, but shall in general include, as necessary, 5' non-transcribed and 5' non-translated sequences involved with the initiation of transcription and translation respectively, such as a TATA box, capping sequence, CAAT sequence, and the like. Especially, such 5' non-transcribed regulatory sequences will include a promoter region which includes a promoter sequence for transcriptional control of the operably joined gene. Regulatory sequences may also include enhancer sequences or upstream activator sequences as desired. The vectors of the invention may optionally include 5' leader or signal sequences. The choice and design of an appropriate vector is within the ability and discretion of one of ordinary skill in the art.

Expression vectors containing all the necessary elements for expression are commercially available and known to those skilled in the art. See, e.g.. Sambrook et al., Molecular Cloning:A Laboratory Manual, Second Edition, Cold Spring Harbor Laboratory Press, 1989. Cells are genetically engineered by the introduction into the cells of heterologous DNA (RNA) encoding a MAGE-3 HLA class II binding peptide. That heterologous DNA (RNA) is placed under operable control of transcriptional elements to permit the expression of the heterologous DNA in the host cell. As described herein, such expression constucts optionally also contain nucleotide sequences which encode endosomal targeting signals, preferably human invariant chain or a targetting fragment thereof

Preferred systems for mRNA expression in mammalian cells are those such as pRc/CMV (available from Invitrogen, Carlsbad, CA) that contain a selectable marker such as a gene that confers G418 resistance (which facilitates the selection of stably transfected cell lines) and the human cytomegalovirus (CMV) enhancer-promoter sequences. Additionally, suitable for expression in primate or canine cell lines is the pCEP4 vector (Invitrogen), which contains an Epstein Barr virus (EBV) origin of replication, facilitating the maintenance of plasmid as a multicopy extrachromosomal element. Another expression vector is the pEF-BOS plasmid containing the promoter of polypeptide Elongation Factor 1 a, which stimulates efficiently transcription *in vitro.* The plasmid is described by Mishizuma and Nagata (Nuc. Acids Res. 18:5322, 1990), and its use in transfection experiments is disclosed by, for example, Demoulin (Mol. Cell. Biol. 16:4710-4716, 1996). Still another preferred expression vector is an adenovirus, described by Stratford-Perricaudet, which is defective for E1 and E3 proteins (J. Clin. Invest. 90:626-630, 1992). The use of the adenovirus as an Adeno.P1A recombinant is disclosed by Warnier et al., in intradermal injection in mice for immunization against P1A (Int. J. Cancer, 67:303-310, 1996).

The invention as described herein has a number of uses, some of which are described herein. The following uses are described for MAGE-3 HLA class II binding peptides but are equally applicable to use of other MAGE family HLA class II binding peptides. First, the invention permits the artisan to diagnose a disorder characterized by expression of a MAGE-3 HLA class II binding peptide. These methods involve determining expression of a MAGE-3 HLA class II binding peptide of the invention, or a complex of a MAGE-3 HLA class II binding peptide of the invention and an HLA class II molecule in a biological sample. The expression of a peptide or complex of peptide and HLA class II molecule can be determined by assaying with a binding partner for the peptide or complex, such as an antibody.

The invention also permits the artisan to treat a subject having a disorder characterized by expression of a MAGE-3 HLA class II binding peptide of the invention. Treatments include administering an agent which enriches in the subject a complex of a MAGE-3 HLA class II binding peptide of the invention and an HLA class II molecule, and administering CD4⁺ T lymphocytes which are specific for such complexes. Agents useful in the foregoing treatments include MAGE-3 HLA class II binding peptides of the invention, compositions of the invention, expression vectors of the invention, cells of the invention and isolated autologous CD4⁺ T Lymphocytes of the invention. The invention also permits an artisan to selectively enrich a population of T lymphocytes for CD4⁺ T lymphocytes specific for a MAGE-3 HLA class II binding peptide of the invention.

The isolation of the MAGE-3 HLA class II binding peptides of the invention also makes it possible to isolate nucleic acids which encode the MAGE-3 HLA class II binding peptides. Nucleic acids can be used to produce *in vitro* or in prokaryotic or eukaryotic host cells the MAGE-3 HLA class II binding peptides. A variety of methodologies well-known to the skilled practitioner can be utilized to obtain isolated MAGE-3 HLA class II binding peptides. For example, an expression vector may be introduced into cells to cause production of the peptides. In another method, mRNA transcripts may be microinjected or otherwise introduced into cells to cause production of the encoded peptides. Translation of mRNA in cell-free extracts such as the reticulocyte lysate system also may be used to produce peptides. Peptides comprising the MAGE-3 HLA class II binding peptide of the invention may also be synthesized *in vitro.* Those skilled in the art also can readily follow known methods for isolating peptides in order to obtain isolated MAGE-3 HLA class II binding peptides. These include, but are not limited to, immunochromotography, HPLC, size-exclusion chromatography, ion-exchange chromatography and immune-affinity chromatography. These isolated MAGE-3 HLA class II binding peptides, or complexes of the peptides and HLA class II molecules, such as an HLA-DRB 1/13 molecule, may be combined with materials such as adjuvants to produce vaccines useful in treating disorders characterized by expression of the MAGE-3 HLA class II binding peptide. In addition, vaccines can be prepared from cells which present the MAGE-3 HLA class II binding peptide/HLA complexes on their surface, such as dendritic cells, B cells, non-proliferative transfectants, etcetera. In all cases where cells are used as a vaccine, these can be cells transfected with coding sequences for one or both of the components necessary to stimulate CD4⁺ lymphocytes, or be cells which already express both molecules without the need for transfection. Vaccines also encompass naked DNA or RNA, encoding a MAGE-3 HLA class II binding peptide or precursor thereof, which may be produced *in vitro* and administered via injection, particle bombardment, nasal aspiration and other methods. Vaccines of the "naked nucleic acid" type have been demonstrated to provoke an immunological response including generation of CTLs specific for the peptide encoded by the naked nucleic acid (Science 259:1745-1748, 1993). Vaccines also include nucleic acids packaged in a virus, liposome or other particle, including polymeric particles useful in drug delivery.

The MAGE-3 HLA class II binding peptides of the invention, as well as complexes of MAGE-3 HLA class II binding peptide and HLA molecule, also may be used to produce antibodies, using standard techniques well known to the art. Standard reference works setting forth the general principles of antibody production include Catty, D., Antibodies, A Practical Approach, Vol. 1, IRL Press, Washington DC (1988); Klein, J., Immunology: The Science of Cell-Non-Cell Discrimination, John Wiley and Sons, New York (1982); Kennett, R., et al., Monoclonal Antibodies, Hybridoma, A New Dimension In Biological Analyses, Plenum Press, New York (1980); Campbell, A., Monoclonal Antibody Technology, in Laboratory Techniques and Biochemistry and Molecular Biology, Vol. 13 (Burdon, R. et al. EDS.), Elsevier Amsterdam (1984); and Eisen, H.N., Microbiology, third edition, Davis, B.D. et al. EDS. (Harper & Rowe, Philadelphia (1980).

The antibodies of the present invention thus are prepared by any of a variety of methods, including administering protein, fragments of protein, cells expressing the protein or fragments thereof and an appropriate HLA class II molecule, and the like to an animal to induce polyclonal antibodies. The production of monoclonal antibodies is according to techniques well known in the art. As detailed herein, such antibodies may be used for example to identify tissues expressing protein or to purify protein. Antibodies also may be coupled to specific labeling agents for imaging or to antitumor agents, including, but not limited to, methotrexate, radioiodinated compounds, toxins such as ricin, other cytostatic or cytolytic drugs, and so forth. Antibodies prepared according to the invention also preferably are specific for the peptide/HLA complexes described herein.

When "disorder" or "condition" is used herein, it refers to any pathological condition where the MAGE-3 HLA class II binding peptides of the invention is expressed. Such disorders include cancers, such as melanomas, squamous cell carcinomas of the head, neck, lung or esophagus, colorectal carcinomas, osteosarcomas, neuroblastomas, non-squamous cell carcinomas of the head or neck, ovarian tumors, lymphocytic leukemias, bladder carcinomas, prostate carcinomas, etc.

Some therapeutic approaches based upon the disclosure are premised on inducing a response by a subject's immune system to MAGE HLA class II binding peptide presenting cells. One such approach is the administration of autologous CD4⁺ T cells specific to the complex of MAGE-3 HLA class II binding peptides of the inventions and an HLA class II molecules to a subject with abnormal cells of the phenotype at issue. It is within the skill of the artisan to develop such CD4⁺ T cells *in vitro.* Generally, a sample of cells taken from a subject, such as blood cells, are contacted with a cell presenting the complex and capable of provoking CD4⁺ T lymphocytes to proliferate. The target cell can be a transfectant, such as a COS cell, or an antigen presenting cell bearing HLA class II molecules, such as dendritic cells or B cells. These transfectants present the desired complex of their surface and, when combined with a CD4⁺ T lymphocyte of interest, stimulate its proliferation. COS cells are widely available, as are other suitable host cells. Specific production of CD4⁺ T lymphocytes is described below. The clonally expanded autologous CD4⁺ T lymphocytes then are administered to the subjects The CD4⁺ T lymphocytes then stimulate the subject's immune response, thereby achieving the desired therapeutic goal.

The foregoing therapy assumes that at least some of the subject's abnormal cells present the relevant HLA/peptide complex. This can be determined very easily, as the art is very familiar with methods for identifying cells which present a particular HLA molecule, as well as how to identify cells expressing DNA of the pertinent sequences, in this case a MAGE-3 sequence.

The foregoing therapy is not the only form of therapy that is available in accordance with the invention. CD4⁺ T lymphocytes can also be provoked *in vivo,* using a number of approaches. One approach is the use of non-proliferative cells expressing the complex. The cells used in this approach may be those that normally express the complex, such as dendritic cells or cells transfected with one or both of the genes necessary for presentation of the complex. Chen et al., (Proc. Natl. Acad. Sci. USA 88: 110-114, 1991) exemplifies this approach, showing the use of transfected cells expressing HPV-E7 peptides in a therapeutic regime. Various cell types may be used. Similarly, vectors carrying one or both of the genes of interest may be used. Viral or bacterial vectors are especially preferred. For example, nucleic acids which encode a MAGE-3 HLA class II binding peptide may be operably linked to promoter and enhancer sequences which direct expresion of the MAGE-3 HLA class II binding peptide in certain tissues or cell types. The nucleic acid may be incorporated into an expression vector. Expression vectors may be unmodified extrachromosomal nucleic acids, plasmids or viral genomes constructed or modified to enable insertion of exogenous nucleic acids, such as those encoding MAGE-3 HLA class II binding peptides. Nucleic acids encoding a MAGE-3 HLA class II binding peptide also may be inserted into a retroviral genome, thereby facilitating integration of the nucleic acid into the genome of the target tissue or cell type. In these systems, the gene of interest is carried by a microorganism, e.g., a Vaccinia virus, retrovirus or the bacteria BCG. and the materials de facto "infect" host cells. The cells which result present the complex of interest, and are recognized by autologous CD4⁺ T cells, which then proliferate.

A similar effect can be achieved by combining a MAGE HLA class II binding peptide with an adjuvant to facilitate incorporation into HLA class II presenting cells *in vivo.* If larger than the HLA class II binding portion, the MAGE-3 HLA class II binding peptide can be processed if necessary to yield the peptide partner of the HLA molecule while the TRA is presented without the need for further processing. Generally, subjects can receive an intradermal injection of an effective amount of the MAGE-3 HLA class II binding peptide. Initial doses can be followed by booster doses, following immunization protocols standard in the art.

A preferred method for facilitating incorporation of MAGE-3 HLA class II binding peptides into HLA class II presenting cells is by attaching (e.g fusing, conjugating) an endosomal targeting signal to a MAGE-3 polypeptide which includes the class II binding peptide. Particularly preferred are MAGE-3 fusion proteins which contain human invariant chain Ii.

Any of the foregoing compositions or protocols can include also MAGE HLA class I binding peptides for induction of a cytolytic T lymphocyte response. For example, as demonstrated below, the MAGE-3 protein can be processed in a cell to produce both HLA class I and HLA class II responses. Several such peptides have been described in U. S. Patents 5,585,461 and 5,591,430 as well as by Gaugler et al. (J. Exp. Med. 179:921-930, 1994), van der Bruggen et al. (Eur. J. Immonol. 24:3038-3043, 1994), and Herman et al. (Immunogenetics 43:377-383, 1996). By administering MAGE-3 peptides which bind HLA class I and class II molecules (or nucleic acid encoding such peptides), an improved immune response may be provided by inducing both T helper cells and T killer cells.

In addition, non-MAGE-3 tumor associated peptides also can be administered to increase immune response via HLA class I and/or class II. It is well established that cancer cells can express more that one tumor associated gene. It is within the scope of routine experimentation for one of ordinary skill in the art to determine whether a particular subject expresses additional tumor associated genes, and then include HLA class I and/or HLA class II binding peptides derived from expression products of such genes in the foregoing MAGE-3 compositions and vaccines.

Especially preferred are nucleic acids encoding a series of epitopes, known as "polytopes". The epitopes can be arranged in sequential or overlapping fashion (*see. e.g.,* Thomson et al., Proc. Natl. Acad. Sci. USA 92:5845-5849, 1995; Gilbert et al., Nature Biotechnol. 15:1280-1284, 1997), with or without the natural flanking sequences, and can be separated by unrelated linker sequences if desired. The polytope is processed to generated individual epitopes which are recognized by the immune system for generation of immune responses.

Thus, for example, MAGE-3 HLA class II binding peptides of the invention can be combined with peptides from other tumor rejection antigens (e.g. by preparation of hybrid nucleic acids or polypeptides) and with MAGE-3 HLA class I binding peptides (some of which are listed below) to form "polytopes". Exemplary tumor associated peptide antigens that can be administered to induce or enhance an immune response are derived from tumor associated genes and encoded proteins including MAGE-1, MAGE-2, MAGE-3, MAGE-4, MAGE-5, MAGE-6. MAGE-7, MAGE-8, MAGE-9, MAGE-10, MAGE-11, GAGE-1, GAGE-2, GAGE-3, GAGE-4, GAGE-5, GAGE-6, BAGE-1, RAGE-1, LB33/MUM-1, PRAME, NAG, MAGE-Xp2, MAGE-Xp3, MAGE-Xp4, tyrosinase, brain glycogen phosphorylase, Melan-A, MAGE-C1, MAGE-C2, NY-ESO-1, SSX-1,SSX-2(HOM-MEL-40), SSX-1, SSX-4, SSX-5, SCP-1 and CT-7. For example, antigenic peptides characteristic of tumors include those listed in Table I below.

**Table I: Exemplary Antigens**

| Gene | MHC | Peptide | Position | SEQ ID NO: |
|---|---|---|---|---|
| MAGE-1 | HLA-A1 | EADPTGHSY | 161-169 | 23 |
| | HLA-Cw16 | SAYGEPRKL | 230-238 | 24 |
| MAGE-3 | HLA-A1 | EVDPIGHLY | 168-176 | 25 |
| | HLA-A2 | FLWGPRALV | 271-279 | 26 |
| | HLA-B44 | MEVDPIGHLY | 167-176 | 27 |
| BAGE | HLA-Cw16 | AARAVFLAL | 2-10 | 28 |
| GAGE-1,2 | HLA-Cw16 | YRPRPRRY | 9-16 | 29 |
| RAGE | HLA-B7 | SPSSNRIRNT | 11-20 | 30 |
| GnT-V | HLA-A2 | VLPDVFIRC(V) | 2-10/11 1 | 31.32 |
| MUM-1 | HLA-B44 | EEKLIVVLF | exon 2/intron | 33 |
| | | EEKLSVVLF (wild type) | | 34 |
| CDK4 | HLA-A2 | ACDPHSGHFV | 23-32 | 35 |
| | | ARDPHSGHFV (wild type) | | 36 |
| β-catenin | HLA-A24 | SYLDSGIHF | 29-37 | 37 |
| | | SYLDSGIHS (wild type) | | 38 |
| Tyrosinase | HLA-A2 | MLLAVLYCL | 1-9 | 39 |
| | HLA-A2 | YMNGTMSQV | 369-377 | 40 |
| | HLA-A2 | YMDGTMSQV | 369-377 | 56 |
| | HLA-A24 | AFLPWHRLF | 206-214 | 41 |
| | HLA-B44 | SEIWRDIDF | 192-200 | 42 |
| | HLA-B44 | YEIWRDIDF | 192-200 | 43 |
| | HLA-DR4 | QNILLSNAPLGPQFP | 56-70 | 44 |
| | HLA-DR4 | DYSYLQDSDPDSFQD | 448-462 | 45 |
| Melan-A^{MART-1} | HLA-A2 | (E)AAGIGILTV | 26/27-35 | 46,47 |
| | HLA-A2 | ILTVILGVL | 32-40 | 48 |
| gp100^{Pmel117} | HLA-A2 | KTWGQYWQV | 154-162 | 49 |
| | HLA-A2 | ITDQVPFSV | 209-217 | 50 |
| | HLA-A2 | YLEPGPVTA | 280-288 | 51 |
| | HLA-A2 | LLDGTATLRL | 457-466 | 52 |
| | HLA-A2 | VLYRYGSFSV | 476-485 | 53 |
| PRAME | HLA-A24 | LYVDSLFFL | 301-309 | 54 |
| MAGE-6 | HLA-Cw16 | KISGGPRISYPL | 292-303 | 55 |
| NY-ESO-1 | HLA-A2 | SLLMWITQCFL | 157-167 | 57 |
| | HLA-A2 | SLLMWITQC | 157-165 | 58 |
| | HLA-A2 | QLSLLMWIT | 155-163 | 59 |

Other examples of HLA class I and HLA- class II binding peptides will be known to one of ordinary skill in the art (for example, see Coulie. Stem Cells 13:393-403. 1995), and can be used in the invention in a like manner as those disclosed herein. One of ordinary skill in the art can prepare polypeptides comprising one or more MAGE-3 peptides and one or more of the foregoing tumor rejection peptides, or nucleic acids encoding such polypeptides, according to standard procedures of molecular biology.

Thus polytopes are groups of two or more potentially immunogenic or immune response stimulating peptides which can be joined together in various arrangements (e.g. concatenated, overlapping). The polytope (or nucleic acid encoding the polytope) can be administered in a standard immunization protocol, e.g. to animals, to test the effectiveness of the polytope in stimulating, enhancing and/or provoking an immune response.

The peptides can be joined together directly or via the use of flanking sequences to form polytopes, and the use of polytopes as vaccines is well known in the art (see, e.g.. Thomson et al., Proc. Acad. Natl. Acad. Sci USA 92(13):5845-5849, 1995; Gilbert et al.. Nature Biotechnol. 15(12):1280-1284, 1997; Thomson et al., J. Immunol. 157(2):822-826, 1996: Tam et al., J. Exp. Med. 171(1):299-306, 1990). For example, Tam showed that polytopes consisting of both MHC class I and class II binding epitopes successfully generated antibody and protective immunity in a mouse model. Tam also demonstrated that polytopes comprising "strings" of epitopes are processed to yield individual epitopes which are presented by MHC molecules and recognized by CTLs. Thus polytopes containing various numbers and combinations of epitopes can be prepared and tested for recognition by CTLs and for efficacy in increasing an immune response.

It is known that tumors express a set of tumor antigens, of which only certain subsets may be expressed in the tumor of any given patient. Polytopes can be prepared which correspond to the different combination of epitopes representing the subset of tumor rejection antigens expressed in a particular patient. Polytopes also can be prepared to reflect a broader spectrum of tumor rejection antigens known to be expressed by a tumor type. Polytopes can be introduced to a patient in need of such treatment as polypeptide structures, or via the use of nucleic acid delivery systems known in the art (see, e.g., Allsopp et al., Eur. J. Immunol. 26(8):1951-1959, 1996). Adenovirus, pox virus, Ty-virus like particles, adeno-associated virus, plasmids, bacteria, etc. can be used in such delivery. One can test the polytope delivery systems in mouse models to determine efficacy of the delivery system. The systems also can be tested in human clinical trials.

As part of the immunization protocols, substances which potentiate the immune response may be administered with nucleic acid or peptide components of a cancer vaccine. Such immune response potentiating compound may be classified as either adjuvants or cytokines. Adjuvants may enhance the immunological response by providing a reservoir of antigen (extracellularly or within macrophages), activating macrophages and stimulating specific sets of lymphocytes. Adjuvants of many kinds are well known in the art; specific examples include MPL (SmithKline Beecham), a congener obtained after purification and acid hydrolysis of *Salmonella minnesota* Re 595 lipopolysaccharide, QS21 (SmithKline Beecham), a pure QA-21 saponin purified from *Quillja saponaria* extract, DQS21, described in PCT application WO96/33739 (SmithKline Beecham), vitamin E and various water-in-oil emulsions prepared from biodegradable oils such as squalene and/or tocopherol. Cytokines are also useful in vaccination protocols as a result of lymphocyte stimulatory properties. Many cytokines useful for such purposes will be known to one of ordinary skill in the art, including interleukin-12 (IL-12) which has been shown to enhance the protective effects of vaccines (Science 268: 1432-1434, 1995), GM-CSF and IL-18.

There are a number of additional immune response potentiating compounds that can be used in vaccination protocols. These include costimulatory molecules provided in either protein or nucleic acid form. Such costimulatory molecules include the B7-1 and B7-2 (CD80 and CD86 respectively) molecules which are expressed on dendritic cells (DC) and interact with the CD28 molecule expressed on the T cell. This interaction provides costimulation (signal 2) to an antigen/MHC/TCR stimulated (signal 1) T cell, increasing T cell proliferation and effector function. B7 also interacts with CTLA4 (CD152) on T cells and studies involving CTLA4 and B7 ligands indicate that the B7-CTLA4 interaction can enhance antitumor immunity and CTL proliferation (Zheng et al., Proc. Nat'l Acad. Sci. USA 95:6284-6289, 1998).

B7 typically is not expressed on tumor cells so they are not efficient antigen presenting cells (APCs) for T cells. Induction of B7 expression would enable the tumor cells to stimulate more efficiently CTL proliferation and effector function. A combination of B7/IL-6/IL-12 costimulation has been shown to induce IFN-gamma and a Th1 cytokine profile in the T cell population leading to further enhanced T cell activity (Gajewski et al., J. Immunol. 154:5637-5648, 1995). Tumor cell transfection with B7 has been discussed in relation to *in vitro* CTL expansion for adoptive transfer immunotherapy by Wang et al. (J.Immunother. 19:1-8, 1996). Other delivery mechanisms for the B7 molecule would include nucleic acid (naked DNA) immunization (Kim et al., Nature Biotechnol. 15:7:641-646, 1997) and recombinant viruses such as adeno and pox (Wendtner et al., Gene Ther. 4:726-735. 1997). These systems are all amenable to the construction and use of expression cassettes for the coexpression of B7 with other molecules of choice such as the antigens or fragment(s) of antigens discussed herein (including polytopes) or cytokines. These delivery systems can be used for induction of the appropriate molecules *in vitro* and for *in vivo* vaccination situations. The use of anti-CD28 antibodies to directly stimulate T cells *in vitro* and *in vivo* could also be considered.

Lymphocyte function associated antigen-3 (LFA-3) is expressed on APCs and some tumor cells and interacts with CD2 expressed on T cells. This interaction induces T cell IL-2 and IFN-gamma production and can thus complement but not substitute, the B7/CD28 costimulatory interaction (Parra et al., J. Immunol., 158:637-642, 1997; Fenton et al., J. Immunother., 21:95-108, 1998).

Lymphocyte function associated antigen-1 (LFA-1) is expressed on leukocytes and interacts with ICAM-1 expressed on APCs and some tumor cells. This interaction induces T cell IL-2 and IFN-gamma production and can thus complement but not substitute, the B7/CD28 costimulatory interaction (Fenton et al 1998). LFA-1 is thus a further example of a costimulatory molecule that could be provided in a vaccination protocol in the various ways discussed above for B7.

Complete CTL activation and effector function requires Th cell help through the interaction between the Th cell CD40L (CD40 ligand) molecule and the CD40 molecule expressed by DCs (Ridge et al., Nature 393:474, 1998; Bennett et al., Nature 393:478, 1998; Schoenberger et al., Nature 393:480, 1998). This mechanism of this costimulatory signal is likely to involve upregulation of B7 and associated IL-6/IL-12 production by the DC (APC). The CD40-CD40L interaction thus complements the signal 1 (antigen/MHC-TCR) and signal 2 (B7-CD28) interactions.

The use of anti-CD40 antibodies to stimulate DC cells directly, would be expected to enhance a response to tumor associated antigens which are normally encountered outside of an inflammatory context or are presented by non-professional APCs (tumor cells). In these situations Th help and B7 costimulation signals are not provided. This mechanism might be used in the context of antigen pulsed DC based therapies or in situations where Th epitopes have not been defined within known tumor associated antigen precursors.

When administered, the therapeutic compositions of the present invention are administered in pharmaceutically acceptable preparations. Such preparations may routinely contain pharmaceutically acceptable concentrations of salt, buffering agents, preservatives, compatible carriers, supplementary immune potentiating agents such as adjuvants and cytokines and optionally other therapeutic agents.

The preparations of the invention are administered in effective amounts. An effective amount is that amount of a pharmaceutical preparation that alone, or together with further doses, stimulates the desired response. In the case of treating cancer, the desired response is inhibiting the progression of the cancer. This may involve only slowing the progression of the disease temporarily, although more preferably, it involves halting the progression of the disease permanently. In the case of inducing an immune response, the desired response is an increase in antibodies or T lymphocytes which are specific for the MAGE-3 immunogen(s) employed. These desired responses can be monitored by routine methods or can be monitored according to diagnostic methods of the invention discussed herein.

Where it is desired to stimulate an immune response using a therapeutic composition of the invention, this may involve the stimulation of a humoral antibody response resulting in an increase in antibody titer in serum, a clonal expansion of cytotoxic lymphocytes, or some other desirable immunologic response. It is believed that doses of immunogens ranging from one nanogram/kilogram to 100 miligrams/kilogram, depending upon the mode of administration, would be effective. The preferred range is believed to be between 500 nanograms and 500 micrograms per kilogram. The absolute amount will depend upon a variety of factors, including the material selected for administration, whether the administration is in single or multiple doses, and individual patient parameters including age, physical condition, size, weight, and the stage of the disease. These factors are well known to those of ordinary skill in the art and can be addressed with no more than routine experimentation.

### Examples

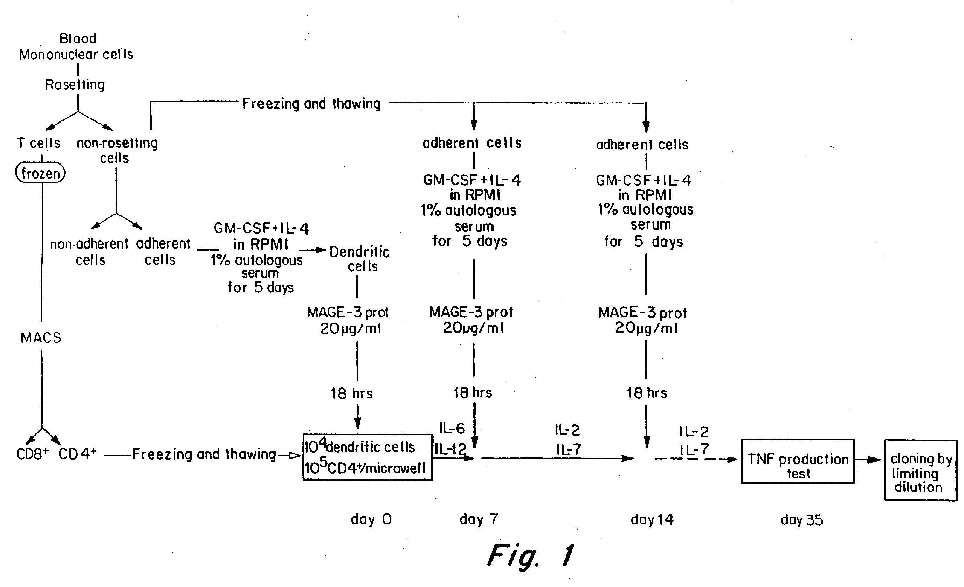

We have identified antigenic peptides encoded by gene MAGE-3 and presented to T cells in the context of HLA class II molecules. The strategy has consisted of loading dendritic cells of normal blood donors with a recombinant MAGE-3 protein and to use these antigen-presenting cells to induce *in vitro* the activation and proliferation of specific CD4 lymphocytes. The protocol is described below (A, B, C) and in Figure 1.

### A. Processing of human blood

Peripheral blood was obtained from the local blood bank (NON CANCER PATIENTS) as standard buffy coat preparations. Peripheral blood mononuclear cells (PBMC) were isolated by centrifugation on Lymphoprep (Nycomed Pharma, Oslo. Norway). In order to minimize contamination of PBMC by platelets, the preparation was first centrifuged for 20 min/1000 rpm at room temperature. After removal of the top 20-25 ml, containing most of the platelets, the tubes were centrifuged for 20 min/1500 rpm at room temperature. PBMC were depleted of T cells by rosetting with 2-aminoethylisothiouronium (Sigma) treated sheep erythrocytes. The lymphocyte-depleted PBMC were left to adhere for 2 hours at 37°C in culture flasks (Falcon) at a density of 2 x 10⁶ cells/ml in RPMI 1640 medium supplemented with L-asparagine (0.24 mM). L-arginine (0.55 mM), L-glutamine (1.5 mM) and 1% autologous serum (complete medium). Non-adherent cells were discarded and adherent cells were cultured in the presence of IL-4 (100 U/ml) and GM-CSF (100 ng/ml) in complete medium. Cultures were fed on day 2 and 4 by removing 5 ml of the medium and adding back fresh medium with IL-4 (100 U/ml) and GM-CSF (100 ng/ml). On day 5, the non-adherent cell population was used as a source of enriched dendritic cells.

Rosetted T cells were treated with NH₄Cl (160 mM) to lyse the sheep erythrocytes, and washed. CD4⁺ T lymphocytes were isolated from rosetted T cells by negative selection using an anti-CD8 monoclonal antibody coupled to magnetic microbeads (Miltenyi Biotech, Germany) by sorting through the Dynal magnet as recommended by the manufacturer.

### B. Cytokines

Human recombinant IL-2 was donated by Biogen (Geneva, Switzerland). Human recombinant IL-4, IL-6 and IL-12 were obtained in our laboratory. Human recombinant IL-7 was purchased from Genzyme (Cambridge, MA). Human recombinant GM-CSF was donated from Sandoz (Sandoz Pharma, Basel, Switzerland). Human recombinant TNF-α was purchased from R & D Systems (Abigdon, UK).

### C. Feeding with protein and mixed lymphocyte-dendritic cells culture

The recombinant His-MAGE-3 protein (MAGE-3 with a His tag) was produced by Smith Kline Corporation Pharmaceutical Company (Rixensart, Belgium) in *E. coli* and purified by standard chromatographic procedures. Autologous dendritic cells were incubated at 37 °C. 5% CO₂, for 18-20 hours in RPM1 medium supplemented with 1% autologous serum. IL-4 (100 U/ml), GM-CSF (100 ng/ml) and TNF-α (1 ng/ml) in the presence of the recombinant His-MAGE-3 protein (20 µg/ml). His-MAGE-3 protein-pulsed dendritic cells were washed and added at 10⁴ per round-bottomed microwell to 10⁵ CD4⁺ T lymphocytes in 200 µl Iscove's medium supplemented with 10% human serum, L-asparagine (0.24 mM). L-arginine (0.55 mM). L-glutamine (1.5 mM) in the presence of IL-6 (1000 U/ml) and IL-12 (10 ng/ml). The CD4⁺ lymphocytes were weekly restimulated with autologous dendritic cells freshly pulsed with the His-MAGE-3 protein and were grown in culture medium supplemented with IL-2 (10 U/ml) and IL-7 (5 ng/ml).

### Example 1: Obtention of CD4 T cell lines and clones specific for MAGE-3

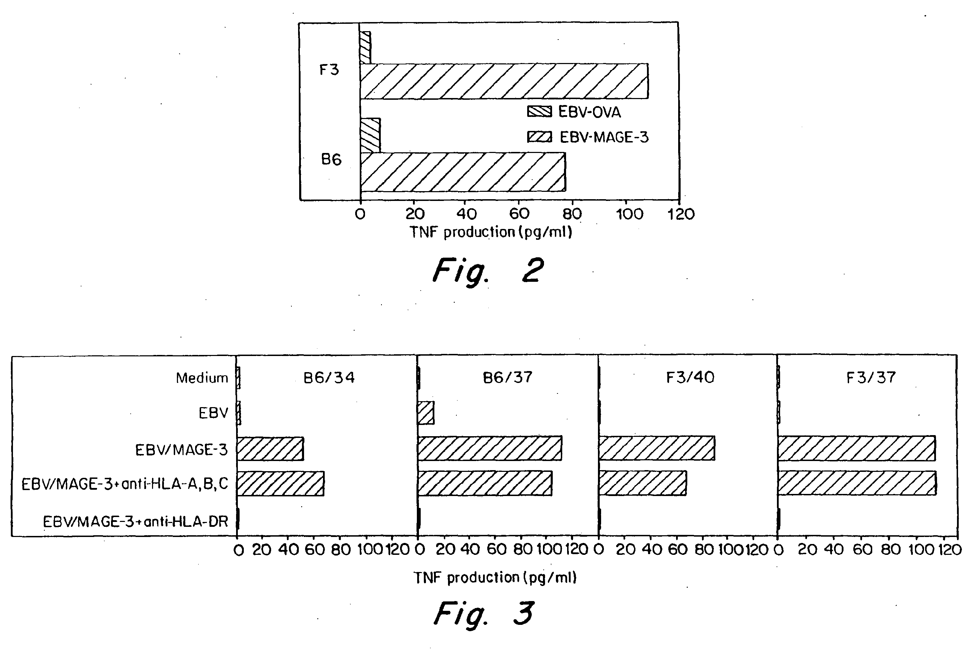

The microcultures that contained proliferating CD4 T cells were assessed 35 days after the start of the culture for their capacity to produce TNF when stimulated with autologous EBV-B cells pulsed with the His-MAGE-3 protein: autologous EBV-B cells were incubated for 18-20 hours in the presence of 20 µg/ml of His-MAGE-3 protein, or Ovalbumin (Sigma) as a negative control. EBV-B cells referred to herein are B cells which were immortalized with Epstein Barr virus. The EBV-B cells were prepared according to art-standard procedures. Protein-pulsed EBV-B cells were washed and added at 5,000 per round-bottomed microwell to 2,500 CD4⁺ T lymphocytes in 150 µl of Iscove's medium supplemented with L-glutamine, L-arginine, L-asparagine, 10% human serum and IL-2 (25 U/ml). After 18-20 hours, supernatants were harvested and assessed for TNF contents by testing their toxicity for TNF-sensitive WEHI 164-13 cells as previously described. The CD4+ T cell lines producing TNF specifically (Fig. 2) were cloned by limiting dilution, using the autologous EBV-B cell line pulsed with exogenous His-MAGE-3 protein as stimulating cells and allogeneic EBV-B cells (LG2-EBV) as feeder cells. CD4 T cell clones were maintained in culture by weekly restimulation with autologous EBV-B cells pulsed with the His-MAGE-3 protein and LG2-EBV in culture medium supplemented with 50 U/ml of IL-2.

CD4 T cell clones were tested for specificity on autologous EBV-B cells pulsed with the exogenous His-MAGE-3 protein: EBV-B cells (500.000/ml) were incubated 18-20 hours at 37°C in the presence 20 µg/ml of the MAGE-3 recombinant protein. Protein-pulsed EBV-B cells were washed and added at 5.000 per round-bottomed microwell to 2.500 CD4+ T lymphocytes in 150 µl of Iscove's medium supplemented with L-glutamine, L-arginine, L-asparagine, 10% human serum and IL-2 (25 U/ml). After 18-20 hours, supernatants were harvested and assessed for TNF and IFN-γ secretion. IFN-γ production was measured using an ELISA assay developed in our laboratory with reagents from Medgenix Diagnostics-Biosourcc (Fleurus, Belgium). Briefly, the assay was a standard ELISA in which IFN-γ antibodies were coated onto the wells of plastic microtiter plates prior to incubation with cell supernatants to determine the amount of IFN-γ produced. Any IFN-γ ELISA assay could be used to measure IFN-γ produced. Several MAGE-3 specific clones were obtained from the B6 line (Fig. 3).

The MAGE-3 epitope is presented to the CD4 clones by HLA-DR molecules (Fig. 3): MAGE-3 -pulsed EBV-B cells were cocultured for 24 hours at 37°C under 8% CO₂ with MAGE-3 specific CD4⁺ clones, in the continuous presence of preservative-free monoclonal antibodies used at a 1/20 dilution. Monoclonal antibody 2B6 (against HLA-DR) abolished the recognition whereas the recognition is unchanged in the presence of monoclonal antibody W6/32 (against HLA-A, B, C).

### Example 2: Identification of the MAGE-3 HLA-DR restricted peptide

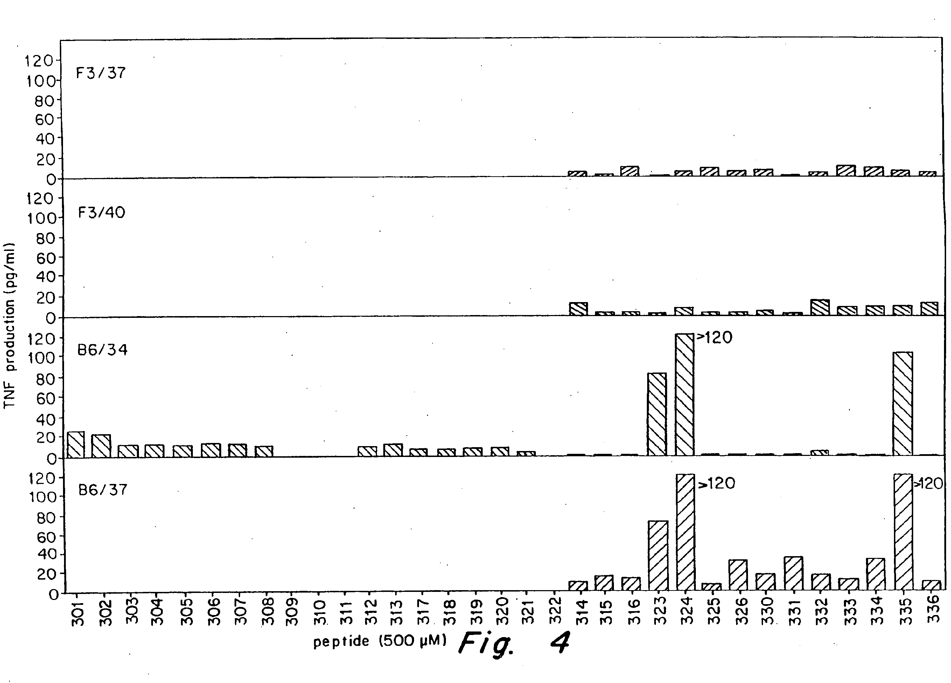

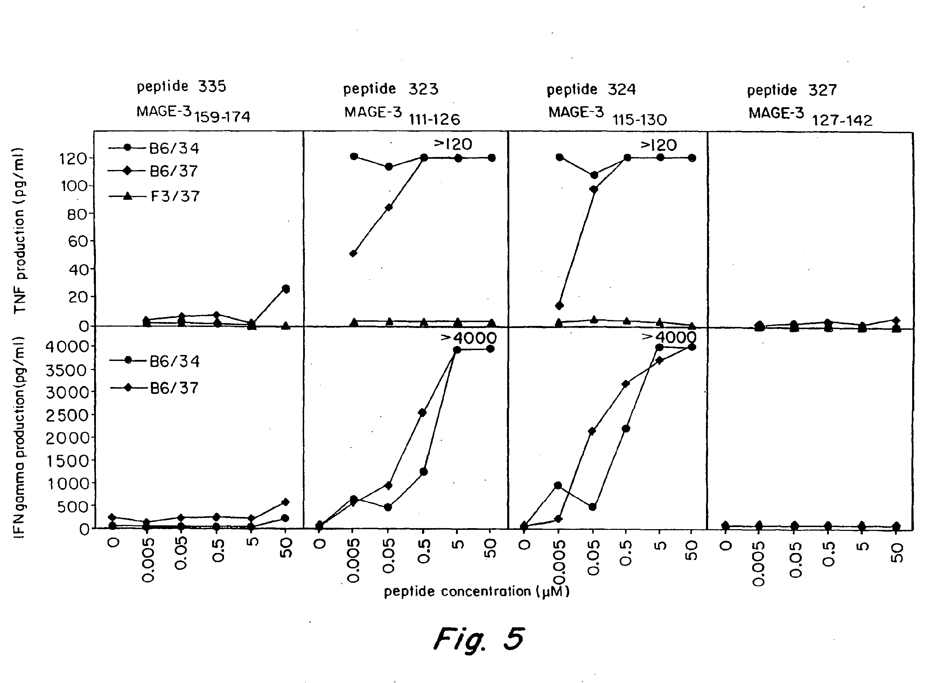

In order to identify the MAG-3 peptides recognized by these CD4 clones, 16 amino acid peptides, corresponding to parts of the MAGE-3 protein sequence were synthesized, loaded on the autologous EBV-B cells and tested for recognition (Fig. 4 and 5). Synthetic peptides were dissolved in DMSO (Merck) and used at a final concentration of 500 µM or 50 µM. EBV-B cells (5,000 per round-bottomed microwell) were incubated 2 hours at 37°C, 8% CO₂ in the presence of the different peptides. CD4⁺ clones were then added at 2,500 cells per well. Assay medium was Iscove's medium supplemented with L-glutamine, L-arginine, L-asparagine. 10% human serum and IL-2 (25 U/ml). After 18-20 hours, supernatants were harvested and assessed for TNF-α and IFN-γ secretion. IFN-γ production was measured using an ELISA test (20-4000 pg/ml) developed in the laboratory with reagents from Medgenix Diagnostics-Biosource (Fleurus, Belgium).

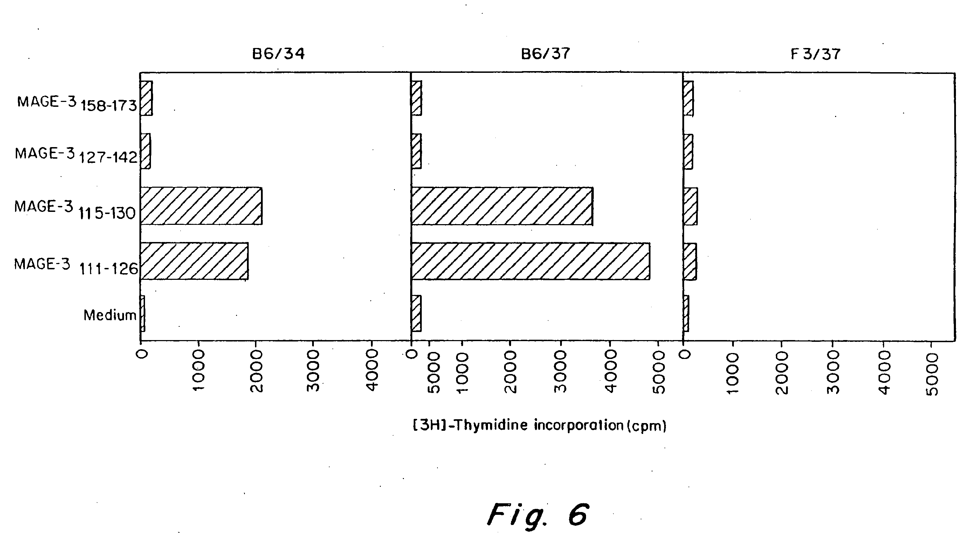

In one set of experiments, the peptides were screened at a non-physiologic concentration of 500 µM. Non-physiologic concentrations of peptide may lead to non-specific activation of T cells clones. Indeed, when used at 500 µM, peptide MAGE-3₁₅₉₋₁₇₄ (Fig. 4- peptide 335; SEQ ID NO:6) induced activation of clones B6/34 and B6/37, but this peptide was not effective in activating these clones when used at 50 µM (Fig. 5). On the contrary, the peptides RKVAELVHFLLLKYRA (MAGE-3₁₁₁₋₁₂₆-Fig. 4-peptides 323; SEQ ID NO:3) and ELVHFLLLKYRAREPV (MAGE-3₁₁₅₋₁₃₀--Fig. 4-peptide 324; SEQ ID NO::4) stimulated specifically TNF-α and IFN-γ production by clones B6/34 and B6/37 when used at physiologic concentrations. These two peptides were also able to induce the proliferation of the B6 clones (Fig. 6).

### Example 3: Determination of the HLA restriction element utilized by MAGE-3 specific CD4⁺ clone B6/34

Cytokine secretion by these CD4⁺ clones in response to autologous EBV-B cell pulsed with the His-MAGE-3 protein is restricted to HLA-DR. To further define the HLA-restriction element utilized by clone B6/37, additional EBV-B cell lines were used for peptide presentation. HLA serotyping of AUMA-EBV, LB 1555-EBV, GERL-EBV revealed that class II molecules shared by all three cell types were limited to HLA-DRB1/1302. Moreover, ADET-EBV was found to present effectively the MAGE-3₁₁₅₋₁₃₀ peptide and the HLA serotyping of these cells was found to be HLA-DRB1/1301. Screening of several other EBV-B cell lines as described above for their ability to stimulate clones B6/34 and B6/37 when pulsed with peptide MAGE-3₁₁₅₋₁₃₀ is performed in order to confirm that both HLA-DRB1/1301 and HLA-DRB1/1302 can present the peptide, or to define other HLA-DRB1/13 presenting molecules.

### Example 4: Determination of the minimal peptide still able to stimulate B6/37 clone

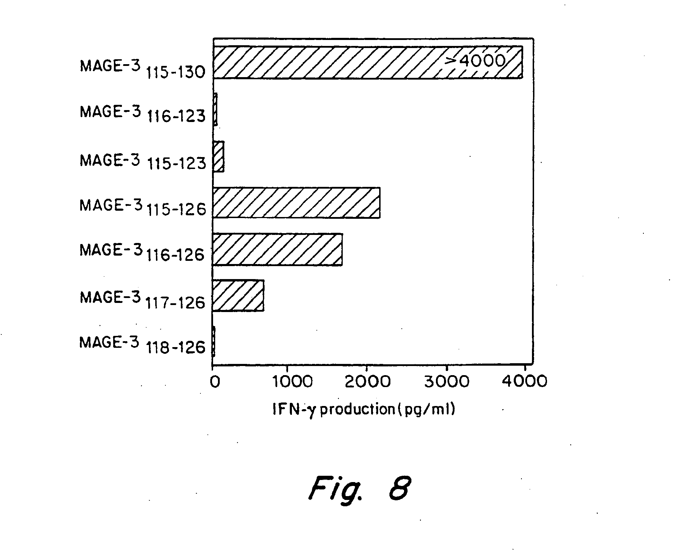

Unlike HLA-class I-restricted peptides, class II-restricted peptides vary considerably in length and can tolerate extensions at both the amino and carboxy termini. We demonstrated that both peptides MAGE-3₁₁₁₋₁₂₆ and MAGE-3₁₁₅₋₁₃₀ stimulated specifically clones B6/34 and B6/37, whereas peptides MAGE-3₁₀₇₋₁₂₂ and MAGE-3₁₁₉₋₁₃₄ were unable to activate these clones. Therefore, the MAGE-3₁₁₅₋₁₂₆ peptide (ELVHFLLLKYRA; SEQ ID NO:9) may be the minimal 12 amino-acids motif necessary for activation of B6/34 and B6/37 clones. As expected, peptide MAGE-3₁₁₅₋₁₂₆ induced significant production of IFN-γ by clone B6/37 (Fig. 8). Shortened peptides having deletions of one residue or more also were prepared. Several of the shortened peptides. e.g. MAGE-3₁₁₆₋₁₂₆ (SEQ ID NO:10) and MAGE-3₁₁₇₋₁₂₆ (SEQ ID NO:11), also induced IFN-γ production by clone B6/37 (Fig. 8), albeit reduced amounts of IFN-γ. MAGE-3₁₁₈₋₁₂₆ (SEQ ID NO: 12) did not induce the production of significant amounts of IFN-γ.

### Example 5: Preparation and use of MAGE-3 fusion proteins

The MAGE-3 protein was expressed in an EBV B cell line MZ2 EBV (HLA A1 DR13) as a fusion protein with the invariant chain (Ii) or with the lysosome-associated membrane protein (LAMP-1) to target the presentation of MAGE-3 derived peptides in HLA class II molecules. Transduction of li MAGE-3 yielded peptide presentation in HLA class II, as measured by the recognition by the CD4 T cell clone LB 1555 CD4 426/B6.37, which reacts with the MAGE-3.DR13 epitope. In addition, expression of Ii MAGE-3 in EBV B cells resulted in peptide presentation in HLA class I, which was determined by the activation of the MAGE-3.A1 specific CTL clone LB 705 434/1. In contrast, expression of the MAGE-3-LAMP-1 fusion protein only marginally enhanced the presentation of MAGE-3 peptide in HLA molecules. Connecting the Ii to MAGE-3 therefore can be used as a vaccine to induce presentation of MAGE-3-derived peptides in both HLA class I and class II.

### Plasmids and cloning of fusion constructs

*MAGE-3:* The MAGE-3 cDNA and polypeptide are set forth as SEQ ID NO:1 and SEQ ID NO:2, respectively.

*Human invariant chain:* The plasmid named IipSV51L containing the human invariant chain encoding cDNA was kindly provided by Dr. J. Pieters (Basel Institute for Immunology. Basel, Switzerland; J. Cell Science 106:831-846, 1993).

*LAMP1:* The plasmid pCMV-sig E7-LAMP1 was kindly provided by Dr. T. Wu (Johns Hopkins University, Baltimore, MD, USA; Proc. Natl. Acad. Sci. USA 92:11671-11675, 1995).

*pMFG:* The plasmid pMFG was kindly provided by Dr. O. Danos (Somatix Therapy Corporation, Alameda, CA, USA).

### Construction of pMFG-MAGE-3:

The MAGE-3 cDNA was transferred to the pMFG vector after the introduction of the appropriate restriction enzyme recognition sites at the 5' and 3' end of the coding sequence. A *Nco*I site was introduced at the 5' site and a *Bgl*II site at the 3' end by PCR using the primers: NcoI-sense: 5'-TTT*CCATGG*CTCTTGAGCAGACIGAGTCAGC-3'(SEQ ID NO: 14) and *Bgl*II-antisense: 5'-CCC*AGATCT*TCACTCTTCCCCCTCTCTC-3' (SEQ ID NO:15) [the recognition sites for *Nco*I and *Bgl*II are in italics]. The PCR product was cloned into a pCR2.1 and sequenced according to standard methods. The *Nco*I-*Bgl*II amplification product was cloned into pMFG opened with the enzymes *Nco*I and *Bam*HI*.*

### Construction of pMFG-Ii.MAGE-3

The cDNA encoding the amino terminal end (i.e. the cytoplasmic tail and the transmembrane region) of the human invariant chain polypeptide (hu-Ii: residues 1 - 80) was amplified by PCR using IipSV51L as template. The following primers were used: hu-Ii sense: 5'-TTT*CCATGG*ATGACCAGCGCGAC-3' (SEQ ID NO:16): and hu-Ii antisense: 5'-TTT*GGATCC*GGAAGCTTCATGCGCAGGTTC-3' (SEQ ID NO:17) [the recognition sites for *Nco*I and *Bam*HI are in italic]. The PCR product was cloned into pCR2.1 and sequenced according to standard methods. The *Nco*I*-Bam*HI amplification product was cloned into pMFG, opened with the enzymes *NcoI* and *Bam*HI resulting in pMFG-Ii.

A *Bgl*II recognition site, replacing the ATG codon and in frame with the *Bam*HI site at the 3' end of the truncated Ii-cDNA, was introduced at the 5' end of the MAGE-3 cDNA by PCR using the primers: *Bgl*II-sense: 5' TTT*AGAT*C*T*TGAGCAGAGGAGTCAGC-3' (SEQ ID NO:18) and *Bgl*II-antisense (SEQ ID NO: 15) [the recognition sites for *BglII* are in italic]. The PCR product (*Bgl*II.MAGE-3.*Bgl*II) was cloned into pCR2.1 and sequenced according to standard methods.

The recombinant plasmid pMFG-Ii was reopened with *Bam*HI and the *Bgl*II.MAGE-3.*Bgl*II amplification product was ligated to the compatible ends. Recombinant plasmids containing the MAGE-3 cDNA in frame and in the right orientation were identified by restriction fragment analysis.

### Construction of pMFG-Sig.MAGE-3.LAMP-1

The cDNAs encoding the signal peptide of the LAMP-1 protein and the transmembrane domain and cytoplasmic tail of LAMP-1 were amplified by PCR using pCMV-sig E7-LAMP1 as template. The primer set for the signal peptide of LAMP-1 was: Sig sense: 5'-CCCC*CCATGG*CGGCCCCCGGC-3'(SEQ ID NO:19) and Sig antisense: 5'-GGG*GGATCC*TCAAAGAGTGCTGA-3' (SEQ ID NO:20) [the recognition sites for *Nco*I and *Bam*HI are in italic]. The *Bam*HI site at the 3' end of this cDNA is in frame with the *Bgl*II site at the 5' end of the *Bgl*II.MAGE-3.*Bgl*II fragment. The amplification product was cloned into pMFG to prepare pMFG-Sig.

The primer set for the amplification of the LAMP1 transmembrane domain and cytoplasmic tail was: LAMP-1 sense: 5'-GGG*GGATCC*TAACAACATGTTGATCCCC-3' (SEQ ID NO:21) and LAMP-1 antisense: 5'-GGG*AGATCTC*TAGATGGTCTGGGTCTGA TAGCCGGC-3' (SEQ ID NO:22) [the recognition sites for *Bam*HI and *Bgl*II are in italic]. The amplification product was sequenced according to standard methods and cloned into pMFG-Sig. resulting in plasmid pMFG-Sig.LAMP-1 with an unique *Bam*HI site at the junction of the signal peptide and the transmembrane sequence. To generate the plasmid pMFG-Sig.MAGE-3.LAMP-1. a *Bgl*II- *Bam*HI fragment isolated from the *Bgl*II.MAGE-3.*Bgl*II cDNA was cloned into pMFG-Sig.LAMP-1 opened with *Bam*HI. This cloning step deleted the 3' end of MAGE-3 encoding amino acids 240 - 314.

### Cell lines, media and reagents

The PhoenixAMPHO cell line (kindly provided by Dr. Nolan, Stanford University School of Medicine, CA, USA) is a high titer amphotropic retrovirus producing cell line that has been generated by stable transfection of 293T cells with a Moloney GagPol-IRES-Lyt 2 construct with an RSV promoter and a pPGK hygro selectable marker. These cells were then stably transfected with the Moloney amphotropic envelope gene driven by a CMV promoter and co-selected with the diphtheria toxin resistance gene (pHED-7). This producer cell line is helper virus free.

PhoenixAMPHO cells were cultured and passaged in 175 cm² flasks in DMEM (Life Technologies, Ghent, Belgium) supplemented with 10% heat inactivated FCS, 2 mM L-glutamine, 100 U/ml penicillin and 100 µg/ml streptomycin.

The MZ2-EBV B cell line was generated from B cells of melanoma patient MZ2 (HLA A1 A29 DR0101 DR1302) by infection with EBV. Likewise, the LG2-EBV B cell line was generated from non-cancer patient LG2 (HLA A24 A32 DR7 DR14). MZ2-MEL.43 is a melanoma cell line from patient MZ2. The EBV transformed B cell lines and MZ2-MEL.43 were cultured in Iscove's modified Dulbecco's (ID) medium supplemented with 10% foetal calf serum (FCS), 0.24 mM L-asparagine, 0.55 mM L-arginine and 1.5 mM L-glutamine (AAG).

The cytotoxic T cell clone LB 705 CTL 434/1 is directed against the MAGE-3.A1 epitope and was generated in a primary culture of CD8⁺ T cells from non-cancer patient LB705 (HLA A1 A2) and irradiated autologous PBL (peripheral blood lymphocytes) pulsed with the MAGE-3.A1 peptide. The CD4 T cell clone LB 1555 CD4 426/B6.37 recognized the MAGE-3.DR13 epitope and was identified by a primary culture of T cells of patient LB 1555 DESA (HLA DR3 DR1302) and autologous monocyte-derived dendritic cells preincubated with purified MAGE-3 protein. The T cell clones were cultured in ID supplemented with 10% heat-inactivated human serum (HS), AAG and 50 U/ml recombinant human IL-2 (rh IL-2) in the presence of irradiated feeder cells (LG2-EBV, pooled human PBL) and specific stimulating cells (MZ2-MEL-43 for CTL 434/1 or DESA-EBV preincubated with the MAGE-3 protein for the CD4 T cell clone 426/B6.37).

### Generation of high titer MAGE 3 encoding recombinant retrovirus

The MAGE-3 encoding retroviral vector plasmids, MFG-MAGE-3, MFG-Ii.MAGE-3. MFG-Sig.MAGE-3.LAMP and MFG-EGFP (encoding enhanced green fluorescent protein reporter), were introduced into the PhoenixAMPHO packaging cells by transfection. The MFG retroviral vector is derived from Moloney murine leukemia virus and is lacking in a drug resistance marker nor does it express any other potential antigenic protein except for the inserted cDNA (Rivière, Proc. Natl Acad. Sci. USA 92:6733-6737, 1995). The transfection procedure is a modification of the calcium phosphate-mediated transfection protocol of Graham and van der Eb (Virology 54:536-539).

Twenty four hours prior to transfection, 10.8x10⁶ PhoenixAMPHO cells were plated in 14 ml cell growth medium in a 75 cm² tissue culture flask (Falcon). After adding the cells, the flask was gently shaken forward and backward to distribute cells evenly about the flask bottom. The cells were incubated at 37°C and 5% CO₂. At the time of transfection, when the cells should have reached a confluence of 70-80%, the medium was removed and was replaced by 14 ml fresh PhoenixAMPHO cell growth medium containing 25 mM **[chloroquine???** -- **Please advise]** (Sigma Chemical Co., St. Louis, MO, USA). A transfection cocktail was prepared in a 50 ml tube by adding 40 µg retroviral vector plasmid DNA to water and diluting to 1575 µl final volume. To this DNA solution 225 µl of 2 M CaCl₂ (Sigma) was added. Then, 1800 µl of 2x HeBS (50mM HEPES, 10 mM KCI, 12 mM dextrose. 280 mM NaCl and 1.5 mM Na₂HPO₄ dissolved in distilled water, filtered through 0.2µ filter and stored at -20°C) was added dropwise to the DNA/CaCl₂ solution by vigorously bubbling for 15 seconds with an automatic pipette. The DNA/CaCl₂/HeBS mix was added immediately and dropwise onto the cells and the flask was gently swirled to ensure uniform mixing of DNA/CaPO₄, particles. The cells were incubated at 37°C / 5% CO₂ for 7 to 9 hours and the chloroquine containing medium was changed for fresh PhoenixAMPHO cell growth medium. Approximately 24 hours prior to the harvest of the retroviral supernatant, the PhoenixAMPHO medium was removed and gently replaced by 9 ml of EBV cell growth medium (Iscove's) containing only 2.5% FCS. The retroviral supernatant was harvested 48 hours following transfection by removing the medium from the cells and filtering through a 0.45 µ filter to remove cell debris. After harvest and filtration, the virus containing medium was kept on ice, aliquoted in appropriate volumes in 15 ml polypropylene tubes and stored at -80°C. The MFG-EGFP transfected PhoenixAMPHO cells were assayed for transfection efficiency by FACS analysis.

### Retroviral transduction of EBV cell lines

The EBV transformed cells were infected by resuspending the cells in an infection cocktail and centrifugation. Target cells were resuspended in 60 mm tissue culture plates (Falcon) at a density of 1.0 x10⁶ cells in 4 ml infection cocktail. The plates were centrifuged for 2 hours at 32°C and 1200 rcf in an IEC centrifuge, rotor type 228. For each plate to be transduced, 4 ml of injection cocktail was prepared by diluting the viral supernatant 1:2 in EBV cell growth medium and adding protamine sulfate (Leo) **[??? -- Please advise]** to a final concentration of 6 µg/ml. Centrifugation was followed by another 2 hours of incubation in a humidified incubator at 37°C and cells were transferred to 4 ml of target cell growth medium. This transduction cycle was carried out immediately after plating the cells and was repeated at 24 and 48 hours. The infected EBV cells were assayed for EGFP reporter gene expression by FACS analysis 24 to 48 hours following the third infection cycle.

### Interferon-γ production assay.

5000 T cells of LB705 CTL 434/1 or 3000 T cells of clone LB1555 CD4 426/B6.37 were washed and cultured overnight in the presence of 5000 retrovirally transduced EBV B cells of MZ2-EBV, or LG2-EBV B cells, in 100 µl ID medium containing 10% HS, AAG and 50 U/ml rh IL-2 in a round-bottom 96 wells plate. All cocultures were performed in triplicate. 50 µl culture supernatant was assayed for the presence of IFN-γ by ELISA (IFN-γ ELISA. Biosource). Briefly, ELISA plates precoated with anti-human IFN-γ Ab were washed and incubated with 50 µl culture supernatant and 50 µl biotinylated anti-human IFN-γ Ab (1:1250 in ID. 10% HS. AAG) for 2 h at room temperature (RT). After three washings the plates were incubated with 50 µl per well horseradish peroxidase conjugated streptavidin (1:3000 in PBS/0.5% BSA) for 30 min at RT, which was detected by TMB substrate, and H₂SO₄ to stop the reaction. The optical density was read at 450 nm. Samples containing 4000 pg/ml IFN-γ and 1:2 dilutions were used as standards.

### Cytotoxicity assay.

1x10⁶ EBV B cells were labeled with 25 µCi Na₂⁵¹CrO₄ for 60-90 min at 37°C. The cells were washed and resuspended at 1 x 10⁴/ml. In control assays, MAGE-3.A1 peptide was added to the cell suspension at a concentration of 1µM. LB705 CTL 434/1 T cells were cultured with 1000 labeled target cells per well in V shaped 96 well plates at 37°C at effector to target cell ratios of 30 to 1 and ten-fold dilutions. After 4h, the chromium release (ER) was measured in an aliquot of 100 µl supernatant. Target cells incubated in medium only or in 1% Triton were taken as minimal (SR) and maximal (MR) ⁵¹Cr release, respectively. The percentage experimental ⁵¹Cr release in the samples was calculated as: (ER-SR/MR-SR) x 100%.

### Recognition of transduced EBV B cell lines by T cell clone LB1555 CD4 426/B6.37.

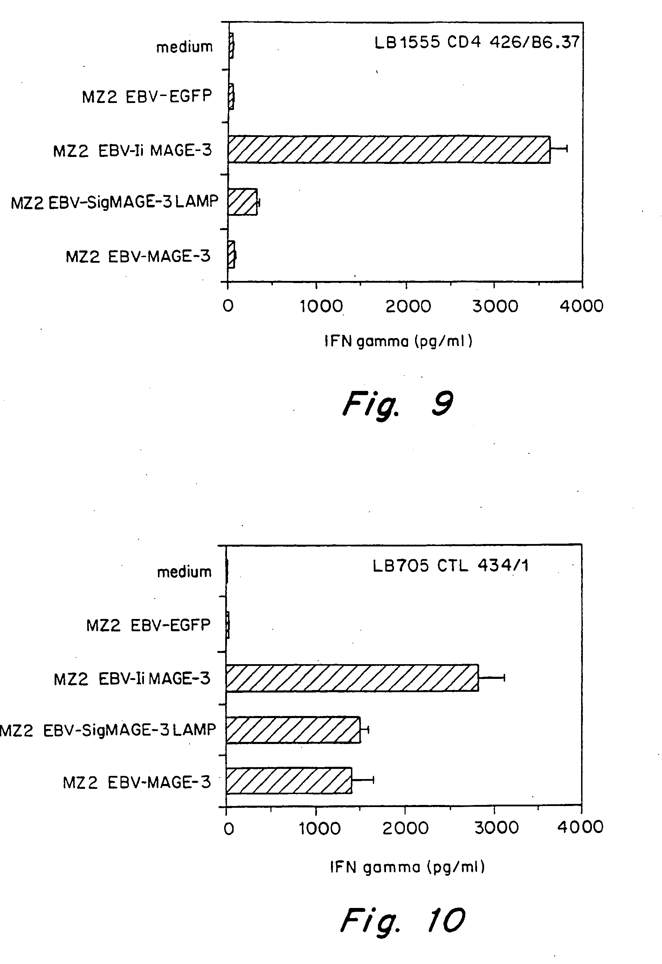

MZ2EBV were transduced with MFG Ii MAGE-3 (MZ2 EBV-Ii MAGE-3). MFG SigMAGE-3 LAMP (MZ2 EBV-SigMAGE-3 LAMP), MFG MAGE-3 (MZ2 EBV-MAGE-3) or with MFG EGFP (MZ2 EBV-EGFP). Transduced cells were cultured overnight in the presence of the CD4T cell clone LB1555 CD4 426/B6.37, which reacts with the MAGE-3.A1 epitope. The T cell clone recognized MZ2-EBV-Ii MAGE-3, as determined by the release of IFN-γ in culture supernatant measured by ELISA (Fig. 9). In contrast, M2 EBV-SigMAGE-3 LAMP only induced a weak production of IFN-γ. The control transfectants, MZ2 EBV-MAGE-3 and MZ2 EBV-EGFP, and LG2 EBV (not shown) were not recognized by the CD4 T cells. These results show that the Ii-MAGE-3 fusion protein is processed for presentation by HLA class II, whereas the MAGE-3 protein alone does not reach the HLA class II antigen presentation pathway.

### Recognition of transduced EBV B cell lines by LB 705 CTL 434/1.

Both MZ2 EBV-Ii MAGE-3 and MZ2 EBV-SigMAGE-3 LAMP were recognized to the same extent by the MAGE-3.A1 specific CTL clone LB 705 CTL 434/1 after overnight coculture (Fig. 10). IFN-γ release in the culture supernatant was measured by ELISA. MZ2 EBV-Ii MAGE-3 elicited a high IFN-γ production by the CTL, clone, indicating that expression of the MAGE-3 protein fused to the li can still lead to processing in the HLA class I pathway.

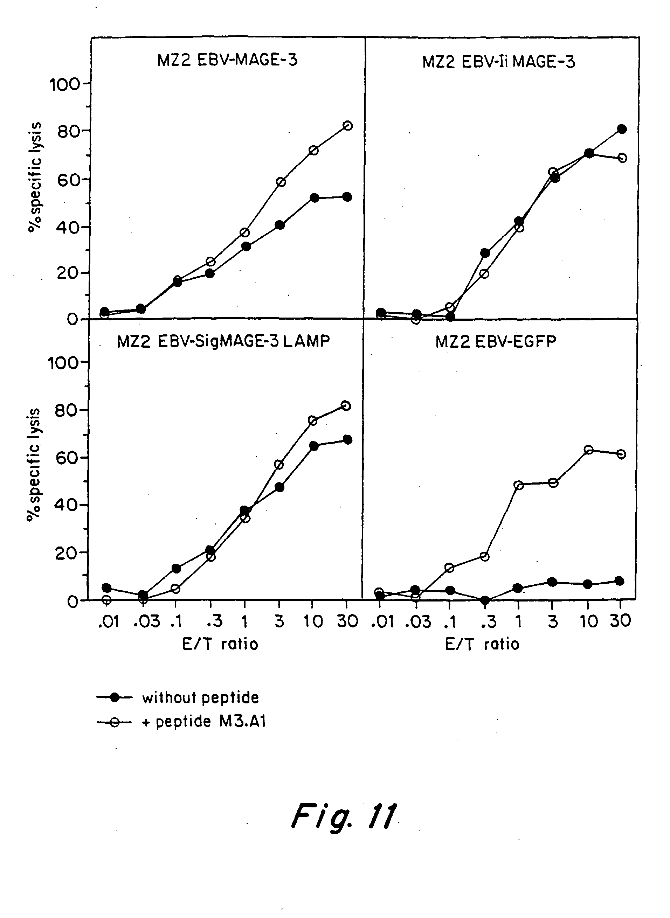

The lysis by LB705 CTL 434/1 of MZ2-EBV retrovirally transduced with MAGE-3, Ii MAGE-3, SigMAGE-3 LAMP or GFP was tested in a 4h 51 Cr release assay at various effector to target cell (E/T) ratios. In parallel, MAGE-3.A1 peptide was added as a positive control for T cell activation. MZ2 EBV-Ii MAGE-3 and MZ2 EBV-SigMAGE-3 LAMP were both lysed by the LB705 CTL 434/1, similar to MZ2 EBV-MAGE-3. The percentage of lysis was equal to the target cell lysis in the presence of the MAGE-3.A1 peptide (Fig. 11).

### Presentation of MAGE-3 derived peptide in HLA class II by MZ2-MEL.43.

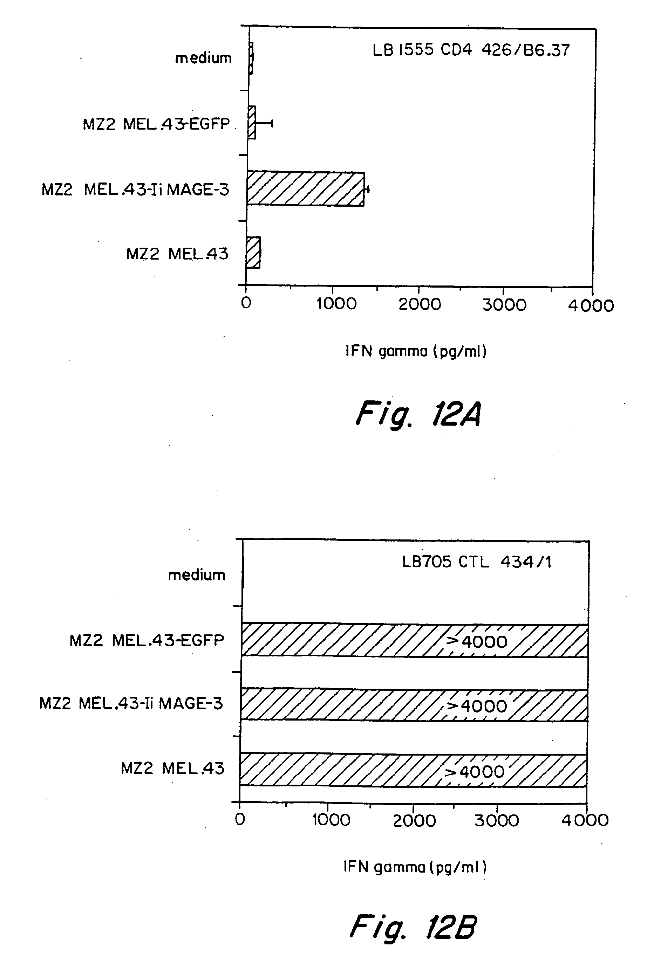

To further confirm to contribution of the Ii to presentation of MAGE-3 peptides in HLA class II molecules, the melanoma cell line MZ2-MEL.43 was transduced with MFG Ii MAGE-3 (MZ2-MEL.43-Ii MAGE-3) or with MFG EGFP (MZ2-MEL.43-EGFP). MZ2-MEL.43 expresses the MAGE-3 protein endogenously, but does not present MAGE-3-derived peptides in HLA class II. However, after transduction of MZ2-MEL.43 with MFG-Ii MAGE-3, it is recognized by the CD4 T cell clone LB1555 CD4 426/B6.37 after overnight coculture (Fig. 12A). IFN-γ release in the culture supernatant was measured by ELISA. This indicates that in contrast to the endogenously expressed. MAGE-3, the Ii MAGE-3 can be processed for presentation in HLA class II. Both parental and transduced MZ2-MEL.43 activated the CTL clone LB705 CTL 434/1 (Fig. 12B), indicating presentation of the MAGE-3 fusion in the HLA class I pathway.

## Claims

1. An isolated MAGE-3 HLA class II-binding peptide comprising the amino acid sequence of SEQ ID NO:11, and that is a fragment of the amino acid sequence of SEQ ID NO: 2.

2. The isolated HLA class II-binding peptide of claim 1 wherein the isolated peptide comprises an amino acid sequence selected from the group consisting of any of SEQ ID NO:3, SEQ ID NO:4, SEQ ID NO:9, and SEQ ID NO:10.

3. The isolated HLA class II-binding peptide of claim 1 wherein the isolated peptide consists of an amino acid sequence selected from the group consisting of any of SEQ ID NO:3, SEQ ID NO:4, SEQ ID NO:9, SEQ ID NO:10 and SEQ ID NO:11.

4. The isolated HLA class II-binding peptide of claim 1, wherein the isolated peptide further comprises an endosomal targeting signal.

5. The isolated HLA class-II binding peptide of claim 4, wherein the endosomal targeting signal comprises an endosomal targeting portion of human invariant chain Ii.

6. The isolated HLA class-II binding peptide of claim 1 wherein the isolated peptide is non-hydrolyzable.

7. The isolated HLA class II-binding peptide of claim 6 wherein the isolated peptide is selected from the group consisting of peptides comprising D-amino acids, peptides comprising a -psi[CH₂NH]-reduced amide peptide bond, peptides comprising a -psi[COCH₂]-ketomethylene peptide bond, peptides comprising a -psi[CH(CN)NH]-(cyanomethylene)amino peptide bond peptides comprising a - psi[CH₂CH(OH))-hydroxyethylene peptide bond, peptides comprising a -psi[CH₂O]-pepti bond, and peptides comprising a -psi[CH₂S]-thiomethylene peptide bond.

8. A composition comprising an isolated MAGE-3 HLA class I-binding peptide and an isolated MAGE-3 HLA class II-binding peptide according to claim 1.

9. The composition of claim 8, wherein the MAGE-3 HLA class I-binding peptide and the MAGE-3 HLA class II-binding peptide are combined as a polytope polypeptide.

10. The composition of claim 9, wherein the isolated MAGE-3 HLA class II-binding peptide consists of an amino acid sequence selected from the group consisting of any of SEQ ID NO:3, SEQ ID NO:4, SEQ ID NO:9, SEQ ID NO:10 and SEQ ID NO:11.

11. The composition of claim 10, wherein the isolated MAGE-3 HLA class II-binding peptide further comprises an endosomal targeting signal.

12. The composition of claim 11, wherein the endosomal targeting signal comprises an endosomal targeting portion of human invariant chain Ii.

13. An isolated nucleic acid encoding a peptide selected from the group consisting of any of the peptide of claim 1, the peptide of claim 2 and the peptide of claim 3.

14. The isolated nucleic acid of claim 13, wherein the nucleic acid comprises SEQ ID NO:13.

15. An expression vector comprising the isolated nucleic acid of claim 13 operably linked to a promoter.

16. The expression vector of claim 15 wherein the nucleic acid comprises SEQ ID NO:13.

17. The expression vector of claim 15 or 16 further comprising a nucleic acid which encodes an HLA-DRB1/13 molecule.

18. A host cell transfected or transformed with an expression vector selected from the group consisting of the expression vector of any of claims 15 to 17.

19. A host cell transfected or transformed with an expression vector selected from the group consisting of the expression vector of claim 15 and the expression vector of claim 16, and wherein the host cell expresses an HLA-DRB1/13 molecule.

20. A method for enriching selectively a population of T lymphocytes with CD4⁺ T lymphocytes specific for a MAGE-3 HLA class II-binding peptide according to claim 1 comprising:
contacting an isolated population of T lymphocytes with an isolated antigen presenting cell which comprises a complex of an HLA class II molecule and an isolated MAGE-3 HLA class II-binding peptide according to claim 1 in an amount sufficient to selectively enrich the isolated population of T lymphocytes with the CD4⁺ T lymphocytes.

21. The method of claim 20 wherein the HLA class II molecule is an HLA-DRB1/13 molecule.

22. The method of claim 20 or 21 wherein the MAGE-3 HLA class II-binding peptide is selected from the group consisting of:
a peptide consisting of the amino acid sequence of SEQ ID NO:3, a peptide consisting of the amino acid sequence of SEQ ID NO:4, a peptide consisting of the amino acid sequence of SEQ ID NO:9, a peptide consisting of the amino acid sequence of SEQ ID NO:10, and a peptide consisting of the amino acid sequence of SEQ ID NO:11.

23. The method of any of claims 20 to 22, wherein the MAGE-3 protein or HLA class II binding peptide thereof comprises an endosomal targeting portion of human invariant chain Ii.

24. An antibody which binds selectively a polypeptide of any of claims 1, 2 or 3.

25. The antibody of claim 24, wherein the antibody is a monoclonal antibody.

26. The antibody of claim 24 or 25, wherein the antibody is an antibody fragment selected from the group consisting of a Fab fragment, a F(ab)₂ fragment or a fragment including a CDR3 region selective for a MAGE-3 HLA class II-binding peptide.

27. A method for diagnosing a disorder **characterized by** expression of MAGE-3 comprising:
contacting a biological sample isolated from a subject with an antibody as defined in any of claims 24 to 26 ; and
determining the interaction between the antibody and the MAGE-3 HLA class II binding peptide as a determination of the disorder.

28. An isolated CD4⁺ T lymphocyte which selectively binds a complex of an HLA class II molecule and an isolated MAGE-3 HLA class II-binding peptide according to claim 1.

29. The isolated CD4⁺ T lymphocyte of claim 28 wherein the HLA class II molecule is an HLA-DRBl/13 molecule.

30. The isolated CD4⁺ T lymphocyte of claim 29 wherein the MAGE-3 HLA class II-binding peptide is selected from the group consisting of:
a peptide consisting of the amino acid sequence of SEQ ID NO:3, a peptide consisting of the amino acid sequence of SEQ ID NO:4, a peptide consisting of the amino acid sequence of SEQ ID NO:9, a peptide consisting of the amino acid sequence of SEQ ID NO:10, and a peptide consisting of the amino acid sequence of SEQ ID NO:11.

31. A method for diagnosing a disorder **characterized by** expression of a MAGE-3 HLA class II-binding peptide according to claim 1 which forms a complex with an HLA class II molecule, comprising:
contacting a biological sample isolated from a subject with an isolated CD4+T lymphocyte as defined in any of claims 28 to 30; and
determining binding between the complex and the lymphocyte as a determination of the disorder.

32. A MAGE-3 HLA Class II - binding peptide as defined in any of claims 1 to 8, a composition as defined in any of claims 8 to 12, an expression vector as defined in any of claims 15 to 17, a cell as defined in claim 18 or 19 or an isolated autologous CD4⁺ T lymphocyte as defined in any of claims 28 to 30 for use in therapy.

33. The MAGE-3 HLA class II-binding peptide, composition, expression vector, cell or CD4⁺ T lymphocyte according to claim 32 wherein the HLA class II molecule is an HLA-DRB1/13 molecule.

34. A method for identifying functional variants of a MAGE-3 HLA class II binding peptide according to claim 1, comprising
selecting a MAGE-3 HLA class II binding peptide of claim 1, an HLA class II binding molecule which binds the MAGE-3 HLA class II binding peptide, and a T cell which is stimulated by the MAGE-3 HLA class II binding peptide presented by the HLA class II binding molecule;
mutating a first amino acid residue of the MAGE-3 HLA class II binding peptide to prepare a variant peptide;
determining the binding of the variant peptide to HLA class II binding molecule and the stimulation of the T cell, wherein binding of the variant peptide to the HLA class II binding molecule and stimulation of the T cell by the variant peptide presented by the HLA class II binding molecule indicates that the variant peptide is a functional variant.

35. The method of claim 34, further comprising the step of comparing the stimulation of the T cell by the MAGE-3 HLA class II binding peptide and the stimulation of the T cell by the functional variant as a determination of the effectiveness of the stimulation of the T cell by the functional variant.

36. A pharmaceutical or vaccine composition comprising an isolated MAGE-3 HLA Class II-binding peptide of any one of claims 1 to 7.

## Patentansprüche

1. Isoliertes MAGE-3 HLA Klasse II-Bindungspeptid, umfassend die Aminosäuresequenz von SEQ ID Nur.:11, und welches ein Fragment der Aminosäuresequenz von SEQ ID Nr.:2 ist.

2. Isoliertes MAGE-3 HLA Klasse II-Bindungspeptid nach Anspruch 1, worin das isolierte Peptid eine Aminosäuresequenz umfasst, ausgewählt aus der Gruppe, bestehend aus einer beliebigen aus SEQ ID Nr.:3, SEQ ID Nr.:4, SEQ ID Nr.:9 und SEQ ID Nur.:10.

3. Isoliertes MAGE-3 HLA Klasse II-Bindungspeptid nach Anspruch 1, worin das isolierte Peptid eine Aminosäuresequenz umfasst, ausgewählt aus der Gruppe, bestehend aus einer beliebigen aus SEQ ID Nr.:3, SEQ ID Nr.:4, SEQ ID Nr.:9, SEQ ID Nr.:10 und SEQ ID Nr.: 11.

4. Isoliertes MAGE-3 HLA Klasse II-Bindungspeptid nach Anspruch 1, worin das isolierte Peptid weiterhin ein endosomales Targeting-Signal umfasst.

5. Isoliertes MAGE-3 HLA Klasse II-Bindungspeptid nach Anspruch 4, worin das endosomale Targeting-Signal einen endosomalen Targeting-Teil der humanen invarianten Kette li umfasst.

6. Isoliertes MAGE-3 HLA Klasse II-Bindungspeptid nach Anspruch 1, worin das isolierte Peptid nicht hydrolysierbar ist.

7. Isoliertes MAGE-3 HLA Klasse II-Bindungspeptid nach Anspruch 6, worin das isolierte Peptid ausgewählt ist aus der Gruppe, bestehend aus Peptiden, umfassend D-Aminosäuren, Peptiden, umfassend eine -psi[CH₂NH]-reduziertes Amid-Peptid-Bindung, Peptide, umfassend eine -psi[COCH_{2]}-Ketomethylen-Peptid-Bindung, Peptide, umfassend eine -psi[CH(CN)NH]-(Cyanomethylen)amino-Peptid-Bindung, Peptide, umfassend eine -psi[CH₂CH(OH)]-Hydroxyethylen-Peptid-Bindung, Peptide, umfassend eine -psi[CH₂O]-Peptid-Bindug und Peptide, umfassend eine -psi[CH₂S]-Thiomethylen-Peptid-Bindung.

8. Zusammensetzung, umfassend ein isoliertes MAGE-3 HLA Klasse I-Bindungspeptid und ein isoliertes MAGE-3 HLA Klasse II-Bindungspeptid nach Anspruch 1.

9. Zusammensetzung nach Anspruch 8, worin das MAGE-3 HLA Klasse 1-Bindungspeptid und das MAGE-3 HLA Klasse II-Bindungspeptid als ein polytopes Polypeptid kombiniert sind.

10. Zusammensetzung nach Anspruch 9, worin das MAGE-3 HLA Klasse II-Bindungspeptid aus einer Aminosäuresequenz besteht, ausgewählt aus der Gruppe, bestehend aus einer aus SEQ ID Nr.:3, SEQ ID Nr.:4, SEQ ID Nr.:9, SEQ ID Nur.:10 und SEQ ID Nr.: 11.

11. Zusammensetzung nach Anspruch 10, worin das MAGE-3 HLA Klasse II-Bindungspeptid weiterhin ein endosomales Targeting-Signal umfasst.

12. Zusammensetzung nach Anspruch 11, worin das endosomale Targeting-Signal einen endosomalen Teil der humanen invarianten Kette li umfasst.

13. Isolierte Nukleinsäure, die für ein Peptid codiert, ausgewählt aus der Gruppe, bestehend aus einem der Peptide nach Anspruch 1, der Peptide nach Anspruch 2 und der Peptide nach Anspruch 3.

14. Isolierte Nukleinsäure nach Anspruch 13, worin die Nukleinsäure SEQ ID Nr.:13 umfasst.

15. Expressionsvektor, umfassend die isolierte Nukleinsäure nach Anspruch 13, operativ verbunden mit einem Promotor.

16. Expressionsvektor nach Anspruch 15, worin die Nukleinsäure SEQ ID Nr.:13 umfasst.

17. Expressionsvektor nach Anspruch 15 oder 16, weiterhin umfassend eine Nukleinsäure, die für ein HLA-DRB1/13-Molekül codiert.

18. Wirtszelle, die transfiziert oder transformiert ist mit einem Expressionsvektor, ausgewählt aus der Gruppe, bestehend aus dem Expressionsvektor nach einem der Ansprüche 15 bis 17.

19. Wirtszelle, die transfiziert oder transformiert ist mit einem Expressionsvektor, ausgewählt aus der Gruppe, bestehend aus dem Expressionsvektor nach Anspruch 15 und dem Expressionsvektor nach Anspruch 16, und worin die Wirtszelle ein HLA-DRB1/13-Molekül exprimiert.

20. Verfahren zum selektiven Anreichern einer Population von T-Lymphozyten mit CD4⁺ T-Lymphozyten, die spezifisch sind für ein MAGE-3 HLA Klasse II-Bindungspeptid nach Anspruch 1, umfassend:
Inkontaktbringen einer isolierten Population von T-Lymphozyten mit einer isolierten Antigen-präsentierenden Zelle, umfassend einen Komplex von einem HLA Klasse II-Molekül und einem isolierten MAGE-3 HLA Klasse II-Bindungspeptid nach Anspruch 1, in einer Menge, die ausreichend ist, um selektiv die isolierte Population von T-Lymphozyten mit den CD4⁺ T-Lymphozyten anzureichern.

21. Verfahren nach Anspruch 20, worin das HLA Klasse II-Molekül ein HLA-DRB1/13-Molekül ist.

22. Verfahren nach Anspruch 20 oder 21, worin das MAGE-3 HLA Klasse II-Bindungspeptid ausgewählt wird aus der Gruppe, bestehend aus:
einem Peptid bestehend aus der Aminosäuresequenz von SEQ ID Nr.:3, einem Peptid, bestehend aus der Aminosäuresequenz von SEQ ID Nr.4, einem Peptid bestehend aus der Aminosäuresequenz von SEQ ID Nr.:9, einem Peptid, bestehend aus der Aminosäuresequenz von SEQ ID Nur.10 und einem Peptid, bestehend aus der Aminosäuresequenz von SEQ ID Nr11.

23. Verfahren nach einem der Ansprüche 20 bis 22, worin das MAGE 3-Protein oder HLA Klasse II-Bindungspeptid davon einen endosomalen Targeting-Teil der humanen invarianten Kette li umfasst.

24. Antikörper, der selektiv ein Polypeptid nach einem der Ansprüche 1, 2 oder 3 bindet.

25. Antikörper nach Anspruch 24, worin der Antikörper ein monoklonaler Antikörper ist.

26. Antikörper nach Anspruch 24 oder 25, worin der Antikörper ein Antikörperfragment ist, ausgewählt aus der Gruppe, bestehend aus einem Fab-Fragment, einem F(ab)₂-Fragment oder einem Fragment, das eine CDR 3-Region aufweist, die selektiv für ein MAGE-3 HLA Klasse II-Bindungspeptid sind.

27. Verfahren zum Diagnostizieren einer Störung, die **gekennzeichnet durch** Expression von MAGE 3, umfassend:
Inkontaktbringen einer von einem Subjekt isolierten biologischen Probe mit einem Antikörper nach einem der Ansprüche 24 bis 26; und
Bestimmen der Wechselwirkung zwischen dem Antikörper und dem MAGE-3 HLA Klasse II-Bindungspeptid als eine Bestimmung der Störung.

28. Isolierter CD4⁺T-Lymphozyt, der selektiv einen Komplex eines HLA Klasse II-Moleküls und eines isolierten MAGE-3 HLA Klasse 11-Bindungspeptids nach Anspruch 1 bindet.

29. Isolierter CD4⁺T-Lymphozyt, nach Anspruch 28, worin das HLA Klasse II-Molekül ein HLA-DRB1/13-Molekül ist.

30. Isolierter CD4⁺T-Lymphozyt nach Anspruch 29, worin das MAGE-3 HLA Klasse II-Bindungspeptid ausgewählt ist aus der Gruppe, bestehend aus:
einem Peptid, bestehend aus der Aminosäuresequenz von SEQ ID Nr.:3, einem Peptid, bestehend aus der Aminosäuresequenz von SEQ ID Nr.4, einem Peptid bestehend aus der Aminosäuresequenz von SEQ ID Nr.:9, einem Peptid, bestehend aus der Aminosäuresequenz von SEQ ID Nur.10 und einem Peptid, bestehend aus der Aminosäuresequenz von SEQ ID Nr.11.

31. Verfahren zum Diagnostizieren einer Störung, die **gekennzeichnet ist durch** die Expression eines MAGE-3 HLA Klasse II-Bindungspeptids nach Anspruch 1, das einen Komplex mit einem HLA Klasse II-Molekül bildet, umfassend:
Inkontaktbringen einer von einem Subjekt isolierten biologischen Probe mit einem isolierten CD4⁺ T-Lymphozyten nach einem der Ansprüche 28 bis 30; und
Bestimmen der Bindung zwischen dem Komplex und dem Lymphozyten als eine Bestimmung der Störung.

32. MAGE-3 HLA Klasse II-Bindungspeptid nach einem der Ansprüche 1 bis 8, Zusammensetzung nach einem der Ansprüche 8 bis 12, Expressionsvektor nach einem der Ansprüche 15 bis 17, Zelle nach einem der Ansprüche 18 oder 19 oder ein isolierter autologer CD4⁺ T-Lymphozyt nach einem der Ansprüche 28 bis 30 zur Verwendung in einer Therapie.

33. MAGE-3 HLA Klasse II-Bindungspeptid, Zusammensetzung, Expressionsvektor, Zelle oder CD4⁺ T-Lymphozyt nach Anspruch 32, worin das HLA-Klasse II-Molekül ein HLA-DRB1/13-Molekül ist.

34. Verfahren zum Identifizieren funktionaler Varianten eines MAGE-3 HLA-Klasse II-Bindungspeptids nach Anspruch 1, umfassend:
Selektieren eines MAGE-3 HLA Klasse II-Bindungspeptids nach Anspruch 1, eines HLA Klasse II-Bindungsmoleküls, das das MAGE-3 HLA Klasse II-Bindungspeptid bindet, und einer T-Zelle, die stimuliert wird durch das MAGE-3 HLA Klasse II-Bindungspeptid, das durch das HLA Klasse II-Bindungsmolekül präsentiert wird;
Mutieren eines ersten Aminosäurerestes des MAGE-3 HLA Klasse II-Bindungspeptids zum Herstellen eines Variantenpeptids;
Bestimmen der Bindung des Variantenpeptids an HLA Klasse II-Bindungsmolekül und der Stimulierung der T-Zelle, worin Bindung des Variantenpeptids an das HLA Klasse II-Bindungsmolekül und
Stimulierung der T-Zelle durch das Variantenpeptid, präsentiert durch das HLA Klasse II-Bindungsmolekül, anzeigt, dass das Variantenpeptid eine funktionale Variante ist.

35. Verfahren nach Anspruch 34, weiterhin umfassend den Schritt des Vergleichens der Stimulation der T-Zelle durch das MAGE-3 HLA Klasse II-Bindungspeptid und der Stimulierung der T-Zelle durch die funktionale Variante als eine Bestimmung der Effizienz der Stimulierung der T-Zelle durch die funktionale Variante.

36. Pharmazeutische oder Impfzusammensetzung, umfassend ein isoliertes MAGE-3 HLA Klasse II-Bindungspeptid nach einem der Ansprüche 1 bis 7.

## Revendications

1. Peptide MAGE-3 isolé de liaison à HLA de classe II, comprenant la séquence d'aminoacides de la SEQ ID N°: 11, et qui est un fragment de la séquence d'aminoacides de la SEQ ID N°: 2.

2. Peptide isolé de liaison à HLA de classe II suivant la revendication 1, ledit peptide isolé comprenant une séquence d'aminoacides choisie dans le groupe consistant en n'importe lesquelles des SEQ ID N°: 3, SEQ ID N°: 4, SEQ ID N°: 9 et SEQ ID N°: 10.

3. Peptide isolé de liaison à HLA de classe II suivant la revendication 1, ledit peptide isolé consistant en une séquence d'aminoacides choisie dans le groupe consistant en n'importe lesquelles des SEQ ID N°: 3, SEQ ID N°: 4, SEQ ID N°: 9, SEQ ID N°: 10 et SEQ ID N°: 11.

4. Peptide isolé de liaison à HLA de classe II suivant la revendication 1, ledit peptide isolé comprenant en outre un signal de ciblage endosomal.

5. Peptide isolé de liaison à HLA de classe II suivant la revendication 4, dans lequel le signal de ciblage endosomal comprend une portion de ciblage endosomal de la chaîne invariante humaine Ii.

6. Peptide isolé de liaison à HLA de classe II suivant la revendication 1, ledit peptide isolé étant non hydrolysable.

7. Peptide isolé de liaison à HLA de classe II suivant la revendication 6, ledit peptide isolé étant choisi dans le groupe consistant en des peptides comprenant des D-aminoacides, des peptides comprenant une liaison peptidique amide réduite -psi[CH₂NH]-, des peptides comprenant une liaison peptidique cétométhylène -psi[COCH₂]-, des peptides comprenant une liaison peptidique (cyanométhylène)amino -psi[CH(CN)NH]-, des peptides comprenant une liaison peptidique hydroxyéthylène -psi[CH₂CH(CO)]-, des peptides comprenant une liaison peptidique -psi[CH₂O]- et des peptides comprenant une liaison peptidique thiométhylène -psi [CH₂S] - .

8. Composition comprenant un peptide MAGE-3 isolé de liaison à HLA de classe I et un peptide MAGE-3 isolé de liaison à HLA de classe II suivant la revendication 1.

9. Composition suivant la revendication 8, dans lequel le peptide MAGE-3 de liaison à HLA de classe I et le peptide MAGE-3 de liaison à HLA de classe II sont combinés sous forme d'un polypeptide polytopique.

10. Composition suivant la revendication 9, dans laquelle le peptide MAGE-3 isolé de liaison à HLA de classe II consiste en une séquence d'aminoacides choisie dans le groupe consistant en n'importe lesquelles des SEQ ID N°: 3, SEQ ID N°: 4, SEQ ID N°: 9, SEQ ID N°: 10 et SEQ ID N°: 11.

11. Composition suivant la revendication 10, dans laquelle le peptide MAGE-3 isolé de liaison à HLA de classe II comprend en outre un signal de ciblage endosomal.

12. Composition suivant la revendication 11, dans laquelle le signal de ciblage endosomal comprend une portion de ciblage endosomal de la chaîne invariante humaine Ii.

13. Acide nucléique isolé codant pour un peptide choisi dans le groupe consistant en le peptide de la revendication 1, le peptide de la revendication 2 et le peptide de la revendication 3.

14. Acide nucléique isolé suivant la revendication 13, ledit acide nucléique comprenant la SEQ ID N°: 13.

15. Vecteur d'expression comprenant l'acide nucléique isolé de la revendication 13, lié de manière fonctionnelle à un promoteur.

16. Vecteur d'expression suivant la revendication 15, dans lequel l'acide nucléique comprend la SEQ ID N°: 13.

17. Vecteur d'expression suivant la revendication 15 ou 16, comprenant en outre un acide nucléique qui code pour une molécule HLA-DRB1/13.

18. Cellule hôte transfectée ou transformée avec un vecteur d'expression choisi dans le groupe consistant en les vecteurs d'expression de n'importe lesquelles des revendications 15 à 17.

19. Cellule hôte transfectée ou transformée avec un vecteur d'expression choisi dans le groupe consistant en le vecteur d'expression de la revendication 15 et le vecteur d'expression de la revendication 16, ladite cellule hôte exprimant une molécule HLA-DRB1/13.

20. Procédé pour enrichir sélectivement une population de lymphocytes T en lymphocytes T CD4⁺ spécifiques d'un peptide MAGE-3 de liaison à HLA de classe II suivant la revendication 1, comprenant:
la mise en contact d'une population isolée de lymphocytes T avec une cellule présentatrice d'antigène isolée qui comprend un complexe d'une molécule de HLA de classe II et d'un peptide MAGE-3 isolé de liaison à HLA de classe II suivant la revendication 1 en une quantité suffisante pour l'enrichissement sélectif de la population isolée de lymphocytes T en lymphocytes T CD4⁺.

21. Procédé suivant la revendication 20, dans lequel la molécule de HLA de classe II est une molécule HLA-DRB1/13.

22. Procédé suivant la revendication 20 ou 21, dans lequel le peptide MAGE-3 de liaison à HLA de classe II est choisi dans le groupe consistant en :
un peptide consistant en la séquence d'aminoacides de la SEQ ID N°: 3, un peptide consistant en la séquence d'aminoacides de la SEQ ID N°: 4, un peptide consistant en la séquence d'aminoacides de la SEQ ID N°: 9, un peptide consistant en la séquence d'aminoacides de la SEQ ID N°: 10 et un peptide consistant en la séquence d'aminoacides de la SEQ ID N°: 11.

23. Procédé suivant l'une quelconque des revendications 20 à 22, dans lequel la protéine MAGE-3 ou son peptide de liaison à HLA de classe II comprend une portion de ciblage endosomal de la chaîne invariante humaine Ii.

24. Anticorps qui se lie sélectivement à un polypeptide de l'une quelconque des revendications 1, 2 et 3.

25. Anticorps suivant la revendication 24, ledit anticorps étant un anticorps monoclonal.

26. Anticorps suivant la revendication 24 ou 25, ledit anticorps étant un fragment d'anticorps choisi dans le groupe consistant en un fragment Fab, un fragment F(ab)₂ et un fragment comprenant une région CDR3 sélective pour un peptide MAGE-3 de liaison à HLA de classe II.

27. Méthode pou diagnostiquer un trouble **caractérisé par** l'expression de MAGE-3, comprenant :
la mise en contact d'un échantillon biologique isolé d'un sujet avec un anticorps tel que défini dans l'une quelconque des revendications 24 à 26 ; et
la détermination de l'interaction entre l'anticorps et le peptide MAGE-3 de liaison à HLA de classe II en tant que détermination du trouble.

28. Lymphocyte T CD4⁺ isolé qui se lie sélectivement à un complexe d'une molécule de HLA de classe II et d'un peptide MAGE-3 isolé de liaison à HLA de classe II suivant la revendication 1.

29. Lymphocyte T CD4⁺ isolé suivant la revendication 28, dans lequel la molécule de HLA de classe II est une molécule HLA-DRB1/13.

30. Lymphocyte T CD4⁺ isolé suivant la revendication 29, dans lequel le peptide MAGE-3 de liaison à HLA de classe II est choisi dans le groupe consistant en :
un peptide consistant en la séquence d'aminoacides de la SEQ ID N°: 3, un peptide consistant en la séquence d'aminoacides de la SEQ ID N°: 4, un peptide consistant en la séquence d'aminoacides de la SEQ ID N°: 9, un peptide consistant en la séquence d'aminoacides de la SEQ ID N°: 10 et un peptide consistant en la séquence d'aminoacides de la SEQ ID N°: 11.

31. Méthode pour diagnostiquer un trouble **caractérisé par** l'expression d'un peptide MAGE-3 de liaison à HLA de classe II suivant la revendication 1 qui forme un complexe avec une molécule de HLA de classe II, comprenant :
la mise en contact d'un échantillon biologique isolé d'un sujet avec un lymphocyte T CD4⁺ isolé tel que défini dans l'une quelconque des revendications 28 à 30 ; et
la détermination de la liaison entre le complexe et le lymphocyte en tant que détermination du trouble.

32. Peptide MAGE-3 de liaison à HLA de classe II tel que défini dans l'une quelconque des revendications 1 à 8, composition telle que définie dans l'une quelconque des revendications 8 à 12, vecteur d'expression tel que défini dans l'une quelconque des revendications 15 à 17, cellule telle que définie dans la revendication 18 ou 19 ou lymphocyte T CD4⁺ autologue isolé tel que défini dans l'une quelconque des revendications 28 à 30, destiné à être utilisé en thérapie.

33. Peptide MAGE-3 de liaison à HLA de classe II, composition, vecteur d'expression, cellule ou lymphocyte T CD4⁺ suivant la revendication 32, dans lequel la molécule de HLA de classe II est une molécule HLA-DRB1/13.

34. Méthode pour identifier des variants fonctionnels d'un peptide MAGE-3 de liaison à HLA de classe II suivant la revendication 1, comprenant :
la sélection d'un peptide MAGE-3 de liaison à HLA de classe II de la revendication 1, d'une molécule de liaison de HLA de classe II qui se lie au peptide MAGE-3 de liaison à HLA de classe II, et d'un lymphocyte T qui est stimulé par le peptide MAGE-3 de liaison à HLA de classe II présenté par la molécule de liaison de HLA de classe II ;
la mutation d'un premier résidu d'aminoacide du peptide MAGE-3 de liaison à HLA de classe II pour préparer un peptide variant ;
la détermination de la liaison du peptide variant à la molécule de liaison de HLA de classe II et de la stimulation du lymphocyte T, où la liaison du peptide variant à la molécule de liaison de HLA de classe II et la stimulation du lymphocyte T par le peptide variant présenté par la molécule de liaison de HLA de classe II indiquant que le peptide variant est un variant fonctionnel.

35. Méthode suivant la revendication 34, comprenant en outre l'étape de comparaison de la stimulation du lymphocyte T par le peptide MAGE-3 de liaison à HLA de classe II et la stimulation du lymphocyte T par le variant fonctionnel en tant que détermination de l'efficacité de la stimulation du lymphocyte T par le variant fonctionnel.

36. Composition pharmaceutique ou composition de vaccin comprenant un peptide MAGE-3 isolé de liaison à HLA de classe II de l'une quelconque des revendications 1 à 7.


### SEQUENCE LISTING

<110> Ludwig Institute for Cancer Research
   Vrije Universiteit Brussels
   Thielemans, Kris
   Heiman, Carlo
   Corthals, Jurgen
   Chaux, Pascal
   Stroobant, Vincent
   Boon-Falleur, Thierry
   van der Bruggen, Pierre
   Luiten, Rosalie
<120> MAGE-3 PEPTIDES PRESENTED BY HLA CLASS II MOLECULES
<130> L0461/7017WO
<140> US 08/928,615
   <141> 1997-09-12
<160> 59
<170> FastSEQ for Windows Version 3.0
<210> 1
   <211> 4204
   <212> DNA
   <213> Homo sapiens
<220>
   <221> CDS
   <222> 2465..3406
<400> 1
<210> 2
   <211> 314
   <212> PRT
   <213> Homo sapiens
<400> 2
   Met Pro Leu Glu Gln Arg Ser Gln His Cys Lys Pro Glu Glu Gly Leu
   1               5                   10                  15
   Glu Ala Arg Gly Glu Ala Leu Gly Leu Val Gly Ala Gln Ala Pro Ala
               20                  25                  30
   Thr Glu Glu Gln Glu Ala Ala Ser Ser Ser Ser Thr Leu Val Glu Val
           35                  40                  45
   Thr Leu Gly Glu Val Pro Ala Ala Glu Ser Pro Asp Pro Pro Gln Ser
       50                  55                  60
   Pro Gln Gly Ala Ser Ser Leu Pro Thr Thr Met Asn Tyr Pro Leu Trp
   65                  70                  75                  80
   Ser Gln Ser Tyr Glu Asp Ser Ser Asn Gln Glu Glu Glu Gly Pro Ser
                   85                  90                  95
   Thr Phe Pro Asp Leu Glu Ser Glu Phe Gln Ala Ala Leu Ser Arg Lys
               100                 105                 110
   Val Ala Glu Leu Val His Phe Leu Leu Leu Lys Tyr Arg Ala Arg Glu
           115                 120                 125
   Pro Val Thr Lys Ala Glu Met Leu Gly Ser Val Val Gly Asn Trp Gln
       130                 135                 140
   Tyr Phe Phe Pro Val Ile Phe Ser Lys Ala Ser Ser Ser Leu Gln Leu
   145                 150                 155                 160
   Val Phe Gly Ile Glu Leu Met Glu Val Asp Pro Ile Gly His Leu Tyr
                   165                 170                 175
   Ile Phe Ala Thr Cys Leu Gly Leu Ser Tyr Asp Gly Leu Leu Gly Asp
               180                 185                 190
   Asn Gln Ile Met Pro Lys Ala Gly Leu Leu Ile Ile Val Leu Ala Ile
           195                 200                 205
   Ile Ala Arg Glu Gly Asp Cys Ala Pro Glu Glu Lys Ile Trp Glu Glu
       210                 215                 220
   Leu Ser Val Leu Glu Val Phe Glu Gly Arg Glu Asp Ser Ile Leu Gly
   225                 230                 235                 240
   Asp Pro Lys Lys Leu Leu Thr Gln His Phe Val Gln Glu Asn Tyr Leu
                   245                 250                 255
   Glu Tyr Arg Gln Val Pro Gly Ser Asp Pro Ala Cys Tyr Glu Phe Leu
               260                 265                 270
   Trp Gly Pro Arg Ala Leu Val Glu Thr Ser Tyr Val Lys Val Leu His
           275                 280                 285
   His Met Val Lys Ile Ser Gly Gly Pro His Ile Ser Tyr Pro Pro Leu
       290                 295                 300
   His Glu Trp Val Leu Arg Glu Gly Glu Glu
   305                 310
<210> 3
   <211> 16
   <212> PRT
   <213> Homo sapiens
<400> 3
   Arg Lys Val Ala Glu Leu Val His Phe Leu Leu Leu Lys Tyr Arg Ala
   1 5 10 15
<210> 4
   <211> 16
   <212> PRT
   <213> Homo sapiens
<400> 4
   Glu Leu Val His Phe Leu Leu Leu Lys Tyr Arg Ala Arg Glu Pro Val
   1 5 10 15
<210> 5
   <211> 16
   <212> PRT
   <213> Homo sapiens
<400> 5
   Arg Glu Pro Val Thr Lys Ala Glu Met Leu Gly Ser Val Val Gly Asn
   1 5 10 15
<210> 6
   <211> 16
   <212> PRT
   <213> Homo sapiens
<400> 6
   Gln Leu Val Phe Gly Ile Glu Leu Met Glu Val Asp Pro Ile Gly His
   1 5 10 15
<210> 7
   <211> 8
   <212> PRT
   <213> Homo sapiens
<400> 7
   Leu Val His Phe Leu Leu Leu Lys
   1 5
<210> 8
   <211> 9
   <212> PRT
   <213> Homo sapiens
<400> 8
   Glu Leu Val His Phe Leu Leu Leu Lys
   1 5
<210> 9
   <211> 12
   <212> PRT
   <213> Homo sapiens
<400> 9
   Glu Leu Val His Phe Leu Leu Leu Lys Tyr Arg Ala
   1 5 10
<210> 10
   <211> 11
   <212> PRT
   <213> Homo sapiens
<400> 10
   Leu Val His Phe Leu Leu Leu Lys Tyr Arg Ala
   1 5 10
<210> 11
   <211> 10
   <212> PRT
   <213> Homo sapiens
<400> 11
   Val His Phe Leu Leu Leu Lys Tyr Arg Ala
   1 5 10
<210> 12
   <211> 9
   <212> PRT
   <213> Homo sapiens
<400> 12
   His Phe Leu Leu Leu Lys Tyr Arg Ala
   1 5
<210> 13
   <211> 30
   <212> DNA
   <213> Homo sapiens
<400> 13
   gttcattttc tgctcctcaa gtatcgagcc 30
<210> 14
   <211> 30
   <212> DNA
   <213> Homo sapiens
<400> 14
   tttccatggc tcttgagcag aggagtcagc 30
<210> 15
   <211> 28
   <212> DNA
   <213> Homo sapiens
<400> 15
   cccagatctt cactcttccc cctctctc 28
<210> 16
   <211> 23
   <212> DNA
   <213> Homo sapiens
<400> 16
   tttccatgga tgaccagcgc gac 23
<210> 17
   <211> 30
   <212> DNA
   <213> Homo sapiens
<400> 17
   tttggatccg gaagcttcat gcgcaggttc 30
<210> 18
   <211> 26
   <212> DNA
   <213> Homo sapiens
<400> 18
   tttagatctt gagcagagga gtcagc 26
<210> 19
   <211> 20
   <212> DNA
   <213> Homo sapiens
<400> 19
   cccccatggc ggcccccggc 20
<210> 20
   <211> 23
   <212> DNA
   <213> Homo sapiens
<400> 20
   gggggatcct caaagagtgc tga 23
<210> 21
   <211> 28
   <212> DNA
   <213> Homo sapiens
<400> 21
   gggggatcct aacaacatgt tgatcccc 28
<210> 22
   <211> 36
   <212> DNA
   <213> Homo sapiens
<400> 22
   gggagatctc tagatggtct gggtctgata gccggc 36
<210> 23
   <211> 9
   <212> PRT
   <213> Homo sapiens
<400> 23
   Glu Ala Asp Pro Thr Gly His Ser Tyr
   1 5
<210> 24
   <211> 9
   <212> PRT
   <213> Homo sapiens
<400> 24
   Ser Ala Tyr Gly Glu Pro Arg Lys Leu
   1 5
<210> 25
   <211> 9
   <212> PRT
   <213> Homo sapiens
<400> 25
   Glu Val Asp Pro Ile Gly His Leu Tyr
   1 5
<210> 26
   <211> 9
   <212> PRT
   <213> Homo sapiens
<400> 26
   Phe Leu Trp Gly Pro Arg Ala Leu Val
   1 5
<210> 27
   <211> 10
   <212> PRT
   <213> Homo sapiens
<400> 27
   Met Glu Val Asp Pro Ile Gly His Leu Tyr
   1 5 10
<210> 28
   <211> 9
   <212> PRT
   <213> Homo sapiens
<400> 28
   Ala Ala Arg Ala Val Phe Leu Ala Leu
   1 5
<210> 29
   <211> 8
   <212> PRT
   <213> Homo sapiens
<400> 29
   Tyr Arg Pro Arg Pro Arg Arg Tyr
   1 5
<210> 30
   <211> 10
   <212> PRT
   <213> Homo sapiens
<400> 30
   Ser Pro Ser Ser Asn Arg Ile Arg Asn Thr
   1 5 10
<210> 31
   <211> 9
   <212> PRT
   <213> Homo sapiens
<400> 31
   Val Leu Pro Asp Val Phe Ile Arg Cys
   1 5
<210> 32
   <211> 10
   <212> PRT
   <213> Homo sapiens
<400> 32
   Val Leu Pro Asp Val Phe Ile Arg Cys Val
   1 5 10
<210> 33
   <211> 9
   <212> PRT
   <213> Homo sapiens
<400> 33
   Glu Glu Lys Leu Ile Val Val Leu Phe
   1 5
<210> 34
   <211> 9
   <212> PRT
   <213> Homo sapiens
<400> 34
   Glu Glu Lys Leu Ser Val Val Leu Phe
   1 5
<210> 35
   <211> 10
   <212> PRT
   <213> Homo sapiens
<400> 35
   Ala Cys Asp Pro His Ser Gly His Phe Val
   1 5 10
<210> 36
   <211> 10
   <212> PRT
   <213> Homo sapiens
<400> 36
   Ala Arg Asp Pro His Ser Gly His Phe Val
   1 5 10
<210> 37
   <211> 9
   <212> PRT
   <213> Homo sapiens
<400> 37
   Ser Tyr Leu Asp Ser Gly Ile His Phe
   1 5
<210> 38
   <211> 9
   <212> PRT
   <213> Homo sapiens
<400> 38
   Ser Tyr Leu Asp Ser Gly Ile His Ser
   1 5
<210> 39
   <211> 9
   <212> PRT
   <213> Homo sapiens
<400> 39
   Met Leu Leu Ala Val Leu Tyr Cys Leu
   1 5
<210> 40
   <211> 9
   <212> PRT
   <213> Homo sapiens
<400> 40
   Tyr Met Asn Gly Thr Met Ser Gln Val
   1 5
<210> 41
   <211> 9
   <212> PRT
   <213> Homo sapiens
<400> 41
   Ala Phe Leu Pro Trp His Arg Leu Phe
   1 5
<210> 42
   <211> 9
   <212> PRT
   <213> Homo sapiens
<400> 42
   Ser Glu Ile Trp Arg Asp Ile Asp Phe
   1 5
<210> 43
   <211> 9
   <212> PRT
   <213> Homo sapiens
<400> 43
   Tyr Glu Ile Trp Arg Asp Ile Asp Phe
   1 5
<210> 44
   <211> 15
   <212> PRT
   <213> Homo sapiens
<400> 44
   Gln Asn Ile Leu Leu Ser Asn Ala Pro Leu Gly Pro Gln Phe Pro
   1 5 10 15
<210> 45
   <211> 15
   <212> PRT
   <213> Homo sapiens
<400> 45
   Asp Tyr Ser Tyr Leu Gln Asp Ser Asp Pro Asp Ser Phe Gln Asp
   1 5 10 15
<210> 46
   <211> 10
   <212> PRT
   <213> Homo sapiens
<400> 46
   Glu Ala Ala Gly Ile Gly Ile Leu Thr Val
   1 5 10
<210> 47
   <211> 9
   <212> PRT
   <213> Homo sapiens
<400> 47
   Ala Ala Gly Ile Gly Ile Leu Thr Val
   1 5
<210> 48
   <211> 9
   <212> PRT
   <213> Homo sapiens
<400> 48
   Ile Leu Thr Val Ile Leu Gly Val Leu
   1 5
<210> 49
   <211> 9
   <212> PRT
   <213> Homo sapiens
<400> 49
   Lys Thr Trp Gly Gln Tyr Trp Gln Val
   1 5
<210> 50
   <211> 9
   <212> PRT
   <213> Homo sapiens
<400> 50
   Ile Thr Asp Gln Val Pro Phe Ser Val
   1 5
<210> 51
   <211> 9
   <212> PRT
   <213> Homo sapiens
<400> 51
   Tyr Leu Glu Pro Gly Pro Val Thr Ala
   1 5
<210> 52
   <211> 10
   <212> PRT
   <213> Homo sapiens
<400> 52
   Leu Leu Asp Gly Thr Ala Thr Leu Arg Leu
   1 5 10
<210> 53
   <211> 10
   <212> PRT
   <213> Homo sapiens
<400> 53
   Val Leu Tyr Arg Tyr Gly Ser Phe Ser Val
   1 5 10
<210> 54
   <211> 9
   <212> PRT
   <213> Homo sapiens
<400> 54
   Leu Tyr Val Asp Ser Leu Phe Phe Leu
   1 5
<210> 55
   <211> 12
   <212> PRT
   <213> Homo sapiens
<400> 55
   Lys Ile Ser Gly Gly Pro Arg Ile Ser Tyr Pro Leu
   1 5 10
<210> 56
   <211> 9
   <212> PRT
   <213> Homo sapiens
<400> 56
   Tyr Met Asp Gly Thr Met Ser Gln Val
   1 5
<210> 57
   <211> 11
   <212> PRT
   <213> Homo sapiens
<400> 57
   Ser Leu Leu Met Trp Ile Thr Gln Cys Phe Leu
   1 5 10
<210> 58
   <211> 9
   <212> PRT
   <213> Homo sapiens
<400> 58
   Ser Leu Leu Met Trp Ile Thr Gln Cys
   1 5
<210> 59
   <211> 9
   <212> PRT
   <213> Homo sapiens
<400> 59
   Gln Leu Ser Leu Leu Met Trp Ile Thr
   1 5